(19) Europäisches Patentamt / European Patent Office / Office européen des brevets

(11) **EP 2 548 512 B1**

(12) **EUROPEAN PATENT SPECIFICATION**

(45) Date of publication and mention
of the grant of the patent:
**01.01.2014 Bulletin 2014/01**

(21) Application number: **11840135.5**

(22) Date of filing: **11.11.2011**

(51) Int Cl.:
*A61B 8/00* *(2006.01)*      *A61B 8/08* *(2006.01)*

(86) International application number:
**PCT/JP2011/076603**

(87) International publication number:
**WO 2012/063976 (18.05.2012 Gazette 2012/20)**

(54) **Ultrasound diagnostic device with the associated method and operation program**

Ultraschalldiagnosevorrichtung mit dem entsprechenden Verfahren und Betriebsprogramm

Dispositif de diagnostic échographique ainsi que les procédé et programme associés

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.11.2010 JP 2010253289**

(43) Date of publication of application:
**23.01.2013 Bulletin 2013/04**

(73) Proprietor: **OLYMPUS MEDICAL SYSTEMS CORP.**
**Tokyo 151-0072 (JP)**

(72) Inventors:
• **MIYAKI, Hironaka**
**Tokyo 151-0072 (JP)**
• **KAMBARA, Tadaaki**
**Tokyo 163-1414 (JP)**
• **WADA, Yasuhiro**
**Tokyo 163-1414 (JP)**

(74) Representative: **Gunzelmann, Rainer**
**Wuesthoff & Wuesthoff**
**Patent- und Rechtsanwälte**
**Schweigerstraße 2**
**81541 München (DE)**

(56) References cited:
JP-A- 2004 049 925      JP-A- 2005 253 827
JP-A- 2007 097 671      JP-A- 2007 524 431
JP-A- 2009 523 059      US-A1- 2004 019 276
US-A1- 2006 079 780      US-A1- 2010 249 590

## Description

Field

The present invention relates to an ultrasonic diagnosis apparatus, an operation method of the ultrasonic diagnosis apparatus, and an operation program of the ultrasonic diagnosis apparatus for enabling determination of tissue characterizations of specimens using ultrasonic sound waves.

Background

Typically, in order to perform screening for breast cancer using ultrasonic sound waves, a technology called ultrasonic elastography is known (for example, see Patent Literature 1). The ultrasonic elastography is a technology which makes use of the fact that cancer tissues or tumor tissues inside a body have different hardness depending on the disease progression or depending on the body nature. In this technology, while continually applying external compression to the screening location, the strain amount or the degree of elasticity of the body tissues at the screening location is measured using ultrasonic sound waves, and the measurement result is displayed in the form of cross-sectional images.

Citation List

Patent Literature

Patent Literature 1: WO/2005/122906

Document US 2006/079780 A1 discloses an ultrasonic imaging apparatus capable of generating ultrasonic images including boundaries between different tissues and regions divided by the boundaries in which property of boundaries and the respective tissues can be distinctly identified. The ultrasonic imaging apparatus includes ultrasonic transducers, a boundary information generating unit, a first image data generating unit, a second image data generating unit, and a tissue property image data generating unit.

Summary

Technical Problem

However, in the abovementioned ultrasonic elastography, there is a problem in that it is difficult to transmit applied pressure to the lower part of a vascular channel, such as a blood vessel or a lymph channel. Therefore, when a tumor is formed near the vascular channel, a border of the tumor becomes indistinct, which makes it difficult to distinguish tumor invasion in the vascular channel. Thus, in the ultrasonic elastography, it is sometimes difficult to distinguish tissue characterization with accuracy.

Moreover, in the ultrasonic elastography, there is another problem in that the reliability of a measurement result is low due to individual differences in pressure or compression speed applied by an examiner when compression is applied to the screening location.

The present invention has been made in view of the above and it is an object of the present invention to provide an ultrasonic diagnosis apparatus, an operation method of the ultrasonic diagnosis apparatus, and an operation program of the ultrasonic diagnosis apparatus capable of distinguishing tissue characterization with accuracy and enhancing the measurement result in terms of reliability.

Solution to Problem

In order to solve the abovementioned problem and achieve the object, an ultrasonic diagnosis apparatus according to the invention transmits ultrasonic sound waves to a specimen and receives ultrasonic sound waves reflected from the specimen, and that determines tissue characterization of the specimen based on the received ultrasonic sound waves. The ultrasonic diagnosis apparatus includes the features of claim 1.

In the ultrasonic diagnosis apparatus according to the invention, the feature data extracting unit includes an approximating unit that performs the approximation with respect to the frequency spectrum calculated by the frequency analyzing unit and extracts pre-correction feature data as feature data prior to performing the attenuation correction; and an attenuation correcting unit that performs the attenuation correction with respect to the pre-correction feature data extracted by the approximating unit, and extracts feature data of the frequency spectrum.

Alternatively, in the ultrasonic diagnosis apparatus according to the invention, the feature data extracting unit includes

an attenuation correcting unit that performs the attenuation correction with respect to the frequency spectrum; and an approximating unit that performs the approximation with respect to the frequency spectrum corrected by the attenuation correcting unit and extracts feature data of the frequency spectrum.

In the ultrasonic diagnosis apparatus according to the invention, greater the reception depth of ultrasonic sound waves, greater is the extent of correction performed by the attenuation correcting unit.

In the ultrasonic diagnosis apparatus according to the invention, the approximating unit performs polynomial approximation with respect to the frequency spectrum by means of regression analysis.

In the ultrasonic diagnosis apparatus according to the invention, the approximating unit performs linear approximation with respect to the frequency spectrum and extracts a plurality of sets of feature data that include at least two components from among a gradient of the linear expression, an intercept of the linear expression, and an intensity that is determined using the gradient, the intercept, and a specific frequency included in the frequency band of the frequency spectrum.

In the ultrasonic diagnosis apparatus according to the invention, the attenuation correcting unit performs correction with respect to at least the gradient and the intensity.

In the ultrasonic diagnosis apparatus according to the invention, the memory unit stores therein the average of each set of feature data present in groups classified on the basis of tissue characterizations of the plurality of known specimens, and the tissue characterization determining unit sets a feature data space, which has at least one of the sets of feature data as component, and determines tissue characterization of the specimen based on the distance in the feature data space from a specimen point, for which coordinates in the feature data space indicate sets of feature data serving as components of the feature data space from among the sets of feature data of the frequency spectrum of the specimen, to a known specimen average point, for which coordinates in the feature data space indicate the averages of sets of feature data serving as components of the feature data space from among the sets of feature data in the groups of the known specimens.

In the ultrasonic diagnosis apparatus according to the invention, the tissue characterization determining unit calculates standard deviation of feature data in a population, which is obtained by adding the feature data of the specimen in the groups divided on the basis of tissue characterizations of the plurality of known specimens, and sets tissue characterization of the specimen to tissue characterization corresponding to a group that has the smallest difference between the standard deviation and standard deviation of feature data in the groups.

The ultrasonic diagnosis apparatus according to the invention further includes: a signal amplifying unit that amplifies reception signals of ultrasonic sound waves received from the specimen; and a B-mode-display image data generating unit that generates B-mode-display image data by converting the amplitude of the reception signals that have been amplified by the signal amplifying unit into luminance. With respect to signals to be output to the B-mode-display image data generating unit, the signal amplifying unit performs amplification by varying gain depending on reception depth, while with respect to signals to be output to the frequency analyzing unit, the signal amplifying unit performs amplification with a constant gain.

In the ultrasonic diagnosis apparatus according to the invention, up to a predetermined reception depth, there is a monotonic increase in the gain with respect to signals to be output to the B-mode-display image data generating unit.

The ultrasonic diagnosis apparatus according to the invention further includes: a B-mode-display image data generating unit that generates B-mode-display image data by converting amplitude of reception signals of ultrasonic sound waves into luminance; a determination-result-display image data generating unit that generates visual information corresponding to the feature data of the specimen and generates determination-result-display image data, which is used in displaying tissue characterization of the specimen, by referring to the visual information that has been generated, the B-mode-display image data generated by the B-mode-display image data, and a determination result of the tissue characterization determining unit; and a display unit that displays an image corresponding to the determination-result-display image data generated by the determination-result-display image data generating unit.

In the ultrasonic diagnosis apparatus according to the invention, the determination-result-display image data contains a tissue characterization weighted image in which the tissue characterization determined by the tissue characterization determining unit is highlighted, and the determination-result-display image data generating unit generates the tissue characterization weighted image by substituting an area determined to be predetermined tissue characterization by the tissue characterization determining unit for a feature data image that has the visual information corresponding to the feature data of the specimen.

In the ultrasonic diagnosis apparatus according to the invention, the determination-result-display image data contains a tissue characterization weighted image in which the tissue characterization determined by the tissue characterization determining unit is highlighted, and the determination-result-display image data generating unit generates the tissue characterization weighted image by substituting an area other than an area determined by the tissue characterization determining unit to be a predetermined tissue characterization for a feature data image that has the visual information corresponding to the feature data of the specimen.

In the ultrasonic diagnosis apparatus according to the invention, the determination-result-display image data contains a tissue characterization weighted image in which the tissue characterization determined by the tissue characterization determining unit is highlighted, and the determination-result-display image data generating unit weights mutually-corresponding pixel values in the B-mode display image and in a feature data image that has the visual information corresponding to the feature data of the specimen, and generates the tissue characterization weighted image that has averages of the weighted pixels values as pixel values.

In the ultrasonic diagnosis apparatus according to the invention, the visual information indicates variables constituting a color space.

An operation method according to the invention is a method of an ultrasonic diagnosis apparatus that transmits ultrasonic sound waves to a specimen and receives ultrasonic sound waves reflected from the specimen, and that determines tissue characterization of the specimen  based on the received ultrasonic sound waves. The operation method includes the steps of claim 15.

An operation program according to the invention is a program of an ultrasonic diagnosis apparatus that transmits ultrasonic sound waves to a specimen and receives ultrasonic sound waves reflected from the specimen, and that determines tissue characterization of the specimen based on the received ultrasonic sound waves. The operation program instructs the ultrasonic diagnosis apparatus to perform the steps of claim 16.

Advantageous Effects of Invention

According to the present invention, approximation is performed with respect to a frequency spectrum that has been obtained by analyzing the frequencies of received ultrasonic sound waves. Then, the feature data of a specimen is extracted by performing attenuation correction by which there is a decrease in the contribution of attenuation, which occurs due to the reception depth and the frequency of the ultrasonic sound waves. Based on the extracted feature data of the specimen and based on the feature data of a plurality of known specimens, the tissue characterization of a predetermined area of the specimen is determined. Hence, without having to make use of the strain amount or the degree of elasticity of the body tissues, it becomes possible to make clear distinction between different tissues. As a result, tissue characterizations can be distinguished with accuracy and the measurement result can be enhanced in terms of reliability.

Brief Description of Drawings

FIG. 1 is a block diagram illustrating a configuration of an ultrasonic diagnosis apparatus according to a first embodiment of the present invention.

FIG. 2 is a diagram illustrating a relationship between gains and reception depths of echo signals for B-mode images.

FIG. 3 is a diagram illustrating a relationship between gains and reception depths of echo signals for processing.

FIG. 4 is a flowchart explaining an overview of the operations performed by the ultrasonic diagnosis apparatus according to the first embodiment of the present invention.

FIG. 5 is a diagram illustrating an example of a B-mode image displayed by a display unit of the ultrasonic diagnosis apparatus according to the first embodiment of the present invention.

FIG. 6 is a flowchart explaining an overview of the operations performed by a frequency analyzing unit of the ultrasonic diagnosis apparatus according to the first embodiment of the present invention.

FIG. 7 is a diagram that schematically illustrates data arrangement of a single acoustic ray.

FIG. 8 is a diagram illustrating an example (first example) of the frequency spectrum calculated by the frequency analyzing unit of the ultrasonic diagnosis apparatus according to the first embodiment of the present invention.

FIG. 9 is a diagram illustrating an example (second example) of the frequency spectrum calculated by the frequency analyzing unit of the ultrasonic diagnosis apparatus according to the first embodiment of the present invention.

FIG. 10 is a diagram illustrating a new straight line  that is determined from the feature data obtained upon performing attenuation correction of the feature data related to a straight line illustrated in FIG. 7.

FIG. 11 is a flowchart explaining an overview of the operations performed by a tissue characterization determining unit of the ultrasonic diagnosis apparatus according to the first embodiment of the present invention.

FIG. 12 is a diagram illustrating an example of the feature data space set by the tissue characterization determining unit of the ultrasonic diagnosis apparatus according to the first embodiment of the present invention.

FIG. 13 is a diagram illustrating a display example of the determination result display image displayed by the display unit of the ultrasonic diagnosis apparatus according to the first embodiment of the present invention.

FIG. 14 is a diagram explaining the effect of attenuation correction performed by the ultrasonic diagnosis apparatus according to the first embodiment.

FIG. 15 is a diagram illustrating a display example (first example) of a tissue characterization weighted image in which a color image is used.

FIG. 16 is a diagram that schematically illustrates a black-and-white image of the image illustrated in FIG. 15.

FIG. 17 is a diagram illustrating a display example (second example) of a tissue characterization weighted image in which a color image is used.

FIG. 18 is a diagram that schematically illustrates a black-and-white image of the image illustrated in FIG. 17.

FIG. 19 is a diagram illustrating a display example (third example) of a tissue characterization weighted image in which a color image is used.

FIG. 20 is a diagram that schematically illustrates a black-and-white image of the image illustrated in FIG. 19.

FIG. 21 is a flowchart explaining an overview of the operations performed by the ultrasonic diagnosis apparatus according to a second embodiment of the present invention.

FIG. 22 is a diagram that schematically illustrates an overview of the attenuation correction performed by the ultrasonic diagnosis apparatus according to the second embodiment.

FIG. 23 is a diagram explaining the overview of the tissue characterization determining operation performed by the tissue characterization determining unit of the ultrasonic diagnosis apparatus according to a fifth embodiment of the present invention.

Description of Embodiments

Exemplary illustrative embodiments of the present invention (hereinafter, referred to as "embodiments") are explained below in detail with reference to the accompanying drawings.

(First embodiment)

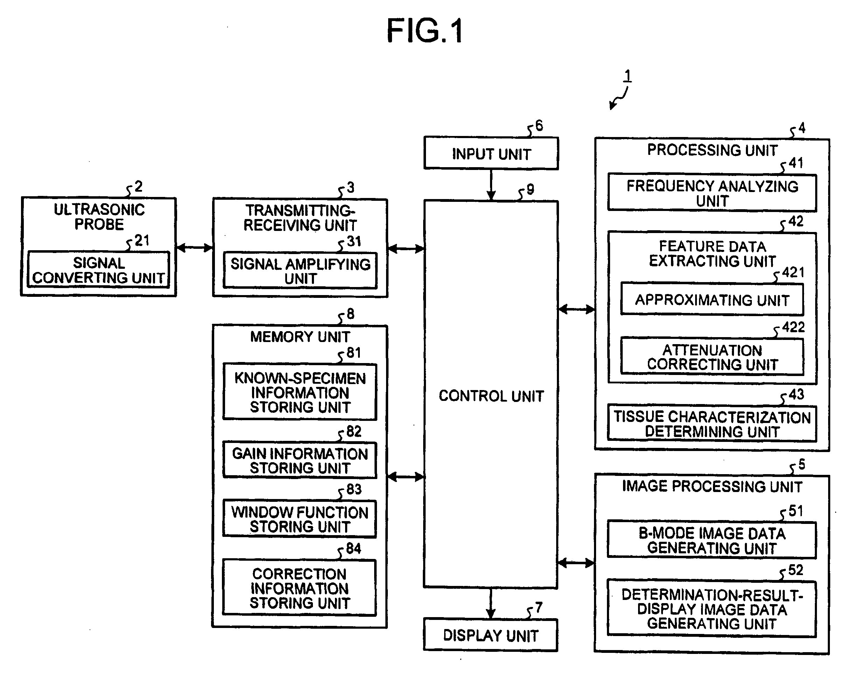

FIG. 1 is a block diagram illustrating a configuration of an ultrasonic diagnosis apparatus according to a first embodiment of the present invention. An ultrasonic diagnosis apparatus 1 illustrated in FIG. 1 is an apparatus for determining tissue characterizations of target specimens for diagnosis using ultrasonic sound waves.

The ultrasonic diagnosis apparatus 1 includes an ultrasonic probe 2 that outputs an ultrasonic pulse to the outside and receives an ultrasonic echo obtained by reflection on the outside; a transmitting-receiving unit 3 that transmits electrical signals to and receives electrical signals from the ultrasonic probe 2; a processing unit 4 that performs predetermined processing on electrical echo signals which are obtained by means of conversion of the ultrasonic echo; an image processing unit 5 that generates image data corresponding to the electrical echo signals which are obtained by means of conversion of the ultrasonic echo; an input unit 6 that is configured with an interface such as a keyboard, a mouse, or a touch-sensitive panel, and that receives input of a variety of information; a display unit 7 that is configured with a liquid crystal display panel or an organic EL display panel, and that displays a variety of information including the images generated by the image processing unit 5; a memory unit 8 that is used to store a variety of information including the information related to tissue characterizations of known specimens; and a control unit 9 that controls the operations of the ultrasonic diagnosis apparatus 1.

The ultrasonic probe 2 converts electrical pulse signals that are received from the transmitting-receiving unit 3 into ultrasonic pulse (acoustic pulse signals), and includes a signal converting unit 21 for converting the ultrasonic echo that is obtained by reflection from an outside specimen into electrical echo signals. Meanwhile, the ultrasonic probe 2 can be configured to have an ultrasonic transducer performing scanning in a mechanical manner or can be configured to have a plurality of ultrasonic transducers performing scanning in an electronic manner.

The transmitting-receiving unit 3 is electrically connected to the ultrasonic probe 2. With that, the transmitting-receiving unit 3 transmits pulse signals to the ultrasonic probe 2 and receives echo signals representing reception signals from the ultrasonic probe 2. More particularly, based on a predetermined waveform and a predetermined transmission timing, the transmitting-receiving unit 3 generates pulse signals and transmits those pulse signals to the ultrasonic probe 2.

The transmitting-receiving unit 3 includes a signal amplifying unit 31 for amplification of echo signals. With respect to echo signals that are used when the image processing unit 5 generates B-mode image data by converting the amplitude of the echo signals into luminance (hereinafter, referred to as "echo signals for B-mode images") and with respect to echo signals that are used by the processing unit 4 to perform processing (hereinafter, referred to as "echo signals for processing"), the signal amplifying unit 31 performs amplification with different gains. More particularly, with respect to the echo signals for B-mode images, the signal amplifying unit 31 performs sensitivity time control (STC) in which the gain is directly proportional to the reception depth of the echo signals. In contrast, with respect to the echo signals for processing, the signal amplifying unit 31 performs amplification with a constant gain irrespective of the reception depth. The signal amplifying unit 31 switches between performing amplification of echo signals for B-mode images and performing amplification of echo signals for processing in the units of frames or in the units of lines.

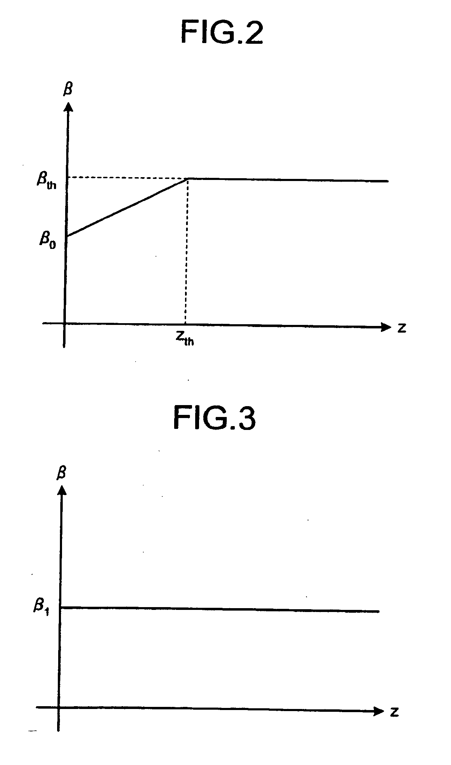

FIG. 2 is a diagram illustrating a relationship between gains and reception depths of echo signals for B-mode images. Herein, with reference to FIG. 2, a reception depth z is calculated based on the elapsed time from the start of receiving ultrasonic sound waves. As illustrated in FIG. 2, when the reception depth z is smaller than a threshold value $z_{th}$, a gain $\beta$ linearly increases from $\beta_0$ to $\beta_{th}$ accompanying the increase in the reception depth z. When the reception depth z is equal to or greater than the threshold value $z_{th}$, the gain $\beta$ remains constant at $\beta_{th}$. The threshold value $z_{th}$ is a value at which most of the ultrasonic signals received from a specimen attenuate, and the noise becomes dominant. Meanwhile, more commonly, when the reception depth z is smaller than the threshold value $z_{th}$, the gain $\beta$ may monotonically increase accompanying the increase in the reception depth z.

FIG. 3 is a diagram illustrating a relationship between gains and reception depths of echo signals for processing. With reference to FIG. 3 too, in an identical manner to the case explained with reference to FIG. 2, the reception depth z is calculated based on the elapsed time from the start of receiving ultrasonic sound waves. As illustrated in FIG. 3, with respect to the echo signals for processing, the signal amplifying unit 31 performs amplification with a constant gain $\beta_1$ irrespective of the reception depth z.

The transmitting-receiving unit 3 performs operations such as filtering with respect to the echo signals amplified by the signal amplifying unit 31, performs A/D conversion of the echo signals to generate digital RF signals, and outputs those digital RF signals. Meanwhile, when the ultrasonic probe 2 is configured to have a plurality of ultrasonic transducers performing scanning in an electronic manner, the transmitting-receiving unit 3 is configured to include a multichannel circuit for performing beam synthesis corresponding to the ultrasonic transducers.

The processing unit 4 includes a frequency analyzing unit 41 that performs frequency analysis of echo signals by carrying out fast Fourier transformation (FFT) with respect to the digital RF signals that are output by the transmitting-receiving unit 3; includes a feature data extracting unit 42 performs attenuation correction and approximation with respect to the frequency spectrum (power spectrum) calculated by the frequency analyzing unit 41 so that there is a decrease in the contribution of attenuation, which occurs due to the reception depth and the frequency of ultrasonic sound waves being propagated, and extracts feature data of a specimen; and includes a tissue characterization determining unit 43 that determines tissue characterization of a predetermined area of the specimen by referring to the feature data extracted by the feature data extracting unit 42.

The frequency analyzing unit 41 calculates a frequency spectrum with respect to each acoustic ray (line data) by performing FFT with respect to an FFT data group having a predetermined volume of data. Depending on the tissue characterization of a specimen, the frequency spectrum demonstrates a different tendency. That is because of the fact that a frequency spectrum has a correlation with the size, the density, and the acoustic impedance of the specimen that serves as a scatterer which scatters the ultrasonic sound waves.

The feature data extracting unit 42 further includes an approximating unit 421, which performs approximation with respect to the frequency spectrum calculated by the frequency analyzing unit 41 and calculates pre-correction feature data that is the feature data prior to performing attenuation correction; and includes an attenuation correcting unit 422, which extracts feature data by performing attenuation correction with respect to the pre-correction feature data obtained by approximation by the approximating unit 421.

The approximating unit 421 performs linear approximation with respect to the frequency spectrum by means of regression analysis so as to extract pre-correction feature data that characterizes the approximated linear expression. More particularly, by means of regression analysis, the approximating unit 421 calculates a gradient $a_0$ and an intercept $b_0$ of the linear expression, as well as calculates the intensity at a specific frequency within the frequency band of the frequency spectrum as the pre-correction feature data. In the first embodiment, it is assumed that, at the central frequency $f_{MID}=(f_{LOW}+f_{HIGH})/2$, the approximating unit 421 calculates $c_0=a_0 f_{MID}+b_0$ as the intensity (Mid-band fit). However, that is only one example. Herein, the intensity indicates any one parameter of parameters such as voltage, power, acoustic pressure, and acoustic energy.

Of the three components of feature data, the gradient $a_0$ has a correlation with the size of the scatterer that scatters the ultrasonic sound waves. Generally, it is thought that larger the scatterer, smaller is the value of the gradient. The intercept $b_0$ has a correlation with the size of the scatterer, the difference in acoustic impedances, and the density (consistency) of the scatterer. More particularly, it is thought that larger the scatterer, greater is the value of the intercept $b_0$; greater the acoustic impedance, greater is the value of the intercept $b_0$; and greater the density (concentration) of the scatterer, greater is the value of the intercept $b_0$. The intensity $c_0$ at the central frequency $f_{MID}$ (hereinafter, simply referred to as "intensity $c_0$") is an indirect parameter derived from the gradient $a_0$ and the intercept $b_0$, and represents the spectrum intensity at the center of the valid frequency band. Thus, it is thought that the intensity $c_0$ has a correlation not only with the size of the scatterer, the difference in acoustic impedances, and the density of the scatterer, but also with the luminosity values of B-mode images to a certain extent. Meanwhile, the approximation polynomial calculated by the feature data extracting unit 42 is not limited to a linear expression. Alternatively, it is also possible to use an approximation polynomial of second-order or more.

The following explanation is given for the correction performed by the attenuation correcting unit 422. An attenuation amount A of ultrasonic sound waves can be expressed as:

$$A = 2\alpha z f \tag{1}$$

where, $\alpha$ represents the attenuation rate, z represents the reception depth of ultrasonic sound waves, and f represents the frequency. As is clear from Equation (1), the attenuation amount A is proportional to the frequency f. Regarding a living body, the specific value of the attenuation rate $\alpha$ is in the range of 0 to 1.0 (dB/cm/MHz) and desirably is in the range of 0.3 to 0.7 (dB/cm/MHz), and is determined according to the organ to be observed. For example, if the organ to be observed is pancreas, then the attenuation rate $\alpha$ is set to 0.6 (dB/cm/MHz). Meanwhile, in the first embodiment, the configuration can also be such that the value of the attenuation rate $\alpha$ can be modified by an input from the input unit 6.

The attenuation correcting unit 422 corrects the pre-correction feature data (the gradient $a_0$, the intercept $b_0$, and the intensity $c_0$), which has been calculated by the approximating unit 421, in the following manner:

$$a = a_0 + 2\alpha z \tag{2}$$

$$b = b_0 \tag{3}$$

$$c = c_0 + 2\alpha z f_{MID} (= a f_{MID} + b) \tag{4}$$

As is clear from Equations (2) and (4) too, greater the reception depth of ultrasonic sound waves, greater is the amount of correction during the correction performed by the attenuation correcting unit 422. Meanwhile, with reference to Equation (3), the correction related to the intercept indicates identical transformation. That is because of the fact that the intercept is a frequency component corresponding to the frequency 0 (Hz) and does not get attenuated.

The tissue characterization determining unit 43 calculates, for each set of feature data of the frequency spectrum extracted by the feature data extracting unit 42, the average and the standard deviation of that set of feature data. Then, by referring to the calculated averages and the calculated standard deviations as well as by referring to the averages and the standard deviations of the sets of feature data of the frequency spectrums of known specimens stored in the memory unit 8, the tissue characterization determining unit 43 determines the tissue characterization of a predetermined area of the specimen. Herein, "predetermined area" indicates an area (hereinafter, referred to as "area of concern") in the image that has been specified by the operator of the ultrasonic diagnosis apparatus 1 upon viewing the images generated by the image processing unit 5. Moreover, for example, "tissue characterization" indicates any one of a cancer, an endocrine tumor, a mucinous tumor, a normal tissue, and a vascular channel. If the specimen is pancreas, then chronic pancreatitis and autoimmune pancreatitis are also considered as tissue characterizations.

The average and the standard deviation of a set of feature data that are calculated by the tissue characterization determining unit 43 reflect the changes at a cellular level such as enlargement or anomaly of the nucleus or reflect the tissue-level changes such as fibrotic growth in the interstitium or substitution of parenchymal tissues with fibers. Thus, depending on the tissue characterization, unique values of the average and the standard deviation are indicated. In consideration of that fact, using the average and the standard deviation of feature data, it becomes possible to correctly determine the tissue characterization of the predetermined area of the specimen.

The image processing unit 5 includes a B-mode image data generating unit 51 that generates B-mode image data from echo signals; and includes a determination-result-display image data generating unit 52 that refers to the data output by the B-mode image data generating unit 51 and by the processing unit 4, and generates determination-result-display image data for displaying the determination result of tissue characterization in the area of concern and for displaying information related to the determination result.

The B-mode image data generating unit 51 generates B-mode image data by performing signal processing on digital signals using a known technology such as bandpass filtering, logarithmic conversion, gain processing, or contrast processing, and by performing data thinning according to the data step width that is decided in accordance to the display range of images in the display unit 7.

The determination-result-display image data generating unit 52 refers to the B-mode image data generated by the B-mode image data generating unit 51, refers to the feature data extracted by the feature data extracting unit 42, and refers to the determination result of the tissue characterization determining unit 43; and generates determination-result-display image data that contains the determination result of tissue characterization in the area of concern and contains a tissue characterization weighted image in which the tissue characterization is highlighted.

The memory unit 8 includes a known-specimen information storing unit 81 that is used to store information on known specimens; includes a gain information storing unit 82 that is used to store information on the gain that is referred to by the signal amplifying unit 31 during amplification; a window function storing unit 83 that is used to store a window function used during frequency analysis performed by the frequency analyzing unit 41; and includes a correction information storing unit 84 that is used to store correction information which is referred to by the attenuation correcting unit 422 while performing operations.

The known-specimen information storing unit 81 is used to store the feature data of frequency spectrums extracted for known specimens and the tissue characterizations of those known specimens in a corresponding manner. In addition, with respect to feature data of the frequency spectrum related to a known specimen; the average and the standard deviation calculated for each group, which is classified on the basis of the tissue characterization of that known specimen, as well as all feature data of that known specimen is stored in the known-specimen information storing unit 81. Herein, it is assumed that the feature data of a known specimen is extracted by performing an identical operation to that performed in the first embodiment. However, the feature data extracting operation for a known specimen need not be performed in the ultrasonic diagnosis apparatus 1. Meanwhile, it is desirable that the information on known specimens stored in the known-specimen information storing unit 81 is reliable in nature regarding tissue characterizations. The gain information storing unit 82 is used to store the relationship between gains and reception depths illustrated in FIG. 2 and FIG. 3. The window function storing unit 83 is used to store at least one window function of the window functions such as Hamming, Hanning, and Blackman. The correction information storing unit 84 is used to store the information related to the conversion of Equations (2) to (4).

Meanwhile, the memory unit 8 is put into practice with a ROM, which is used to store in advance operating programs of the ultrasonic diagnosis apparatus 1 according to the first embodiment and to store programs for running a predetermined OS; and with a RAM, which is used to store operating parameters and data of each operation.

In the ultrasonic diagnosis apparatus 1 having the abovementioned functional configuration, the constituent elements other than the ultrasonic probe 2 are put into practice with a computer that includes a CPU for performing processing and control. The CPU in the ultrasonic diagnosis apparatus 1 reads, from the memory unit 8, the information and various programs including the operating programs of the ultrasonic diagnosis apparatus 1; and performs processing related to the operation method of the ultrasonic diagnosis apparatus 1 according to the first embodiment.

The operating programs of the ultrasonic diagnosis apparatus 1 according to the first embodiment can also be recorded in a computer readable recording medium such as a hard disk, a flash memory, a CD-ROM, a DVD-ROM, or a flexible disk for the purpose of distribution.

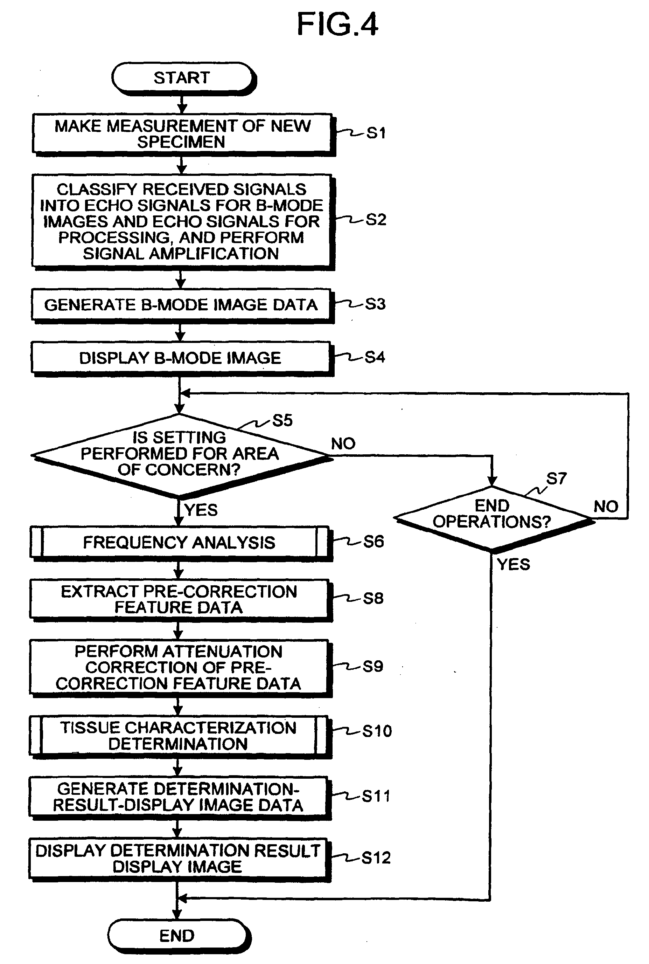

FIG. 4 is a flowchart explaining an overview of the operations performed by the ultrasonic diagnosis apparatus 1 having the configuration explained above. With reference to FIG. 4, firstly, the ultrasonic diagnosis apparatus 1 makes a measurement of a new specimen using the ultrasonic probe 2 (Step S1).

Then, the signal amplifying unit 31 receives echo signals from the ultrasonic probe 2 and performs amplification by classifying the received echo signals into echo signals for B-mode images and echo signals for processing (Step S2). Herein, the signal amplifying unit 31 performs amplification based on the relationship between gains and reception depths as illustrated in FIG. 2 and FIG. 3. Meanwhile, the timing of performing amplification of echo signals for B-mode images and the timing of performing amplification of echo signals for processing can be switched in the units of frames or in the units of lines.

Subsequently, the B-mode image data generating unit 51 generates B-mode image data using the echo signals for B-mode images output by the transmitting-receiving unit 3 (Step S3).

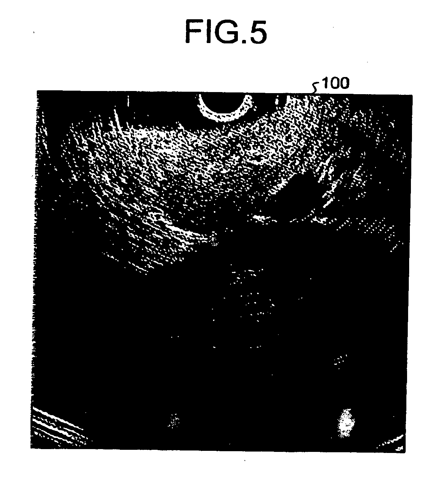

Then, the control unit 9 performs control so that the display unit 7 displays a B-mode image corresponding to the B-mode image data generated by the B-mode image data generating unit 51 (Step S4). FIG. 5 is a diagram illustrating an example of a B-mode image displayed by the display unit 7. A B-mode image 100 illustrated in FIG. 5 is a grayscale image in which variables R (red), G (green), and B (blue), which are variables when the RGB color system is adopted as the color space, have identical values.

Subsequently, if settings for the area of concern are performed via the input unit 6 (Yes at Step S5), the frequency analyzing unit 41 performs frequency analysis by means of FFT and calculates a frequency spectrum (Step S6). At Step S6, it is also possible to set the entire area of an image as the area of concern. Meanwhile, if settings for the area of concern are not yet performed (No at Step S5) but if an instruction to end operations is input via the input unit 6 (Yes at Step S7); then the ultrasonic diagnosis apparatus 1 ends the operations. In contrast, neither settings for the area of concern are performed (No at Step S5) nor an instruction to end operations is input via the input unit 6 (No at Step S7), then the system control returns to Step S5.

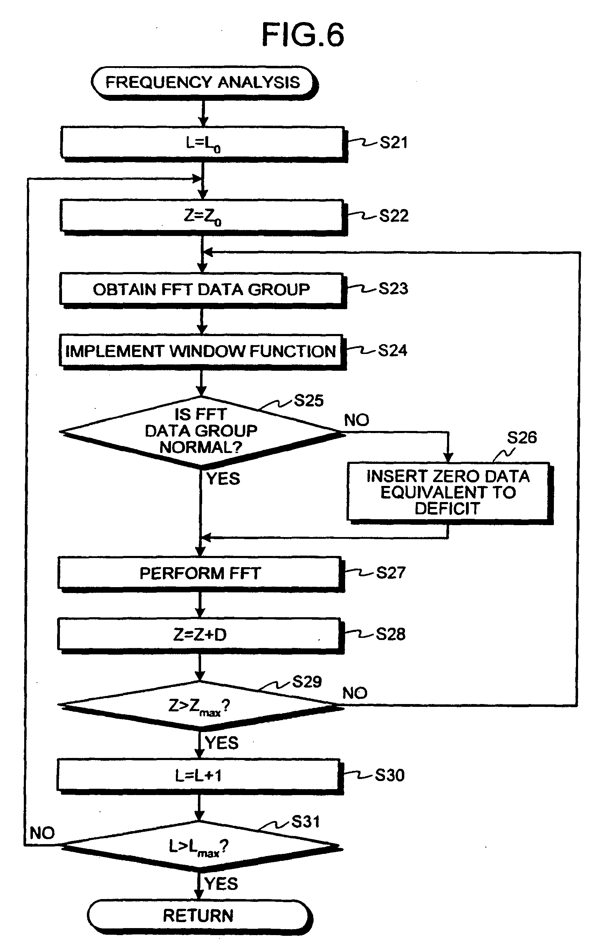

Herein, the operation performed by the frequency analyzing unit 41 (Step S6) is explained in detail with reference to a flowchart illustrated in FIG. 6. Firstly, the frequency analyzing unit 41 sets an acoustic ray number L of the acoustic ray to be initially analyzed to an initial value $L_0$ (Step S21). The initial value $L_0$ can be assigned, for example, to the acoustic ray received at the start by the transmitting-receiving unit 3 or to the acoustic ray corresponding to the border position on any one of the left and right sides of the area of concern set via the input unit 6.

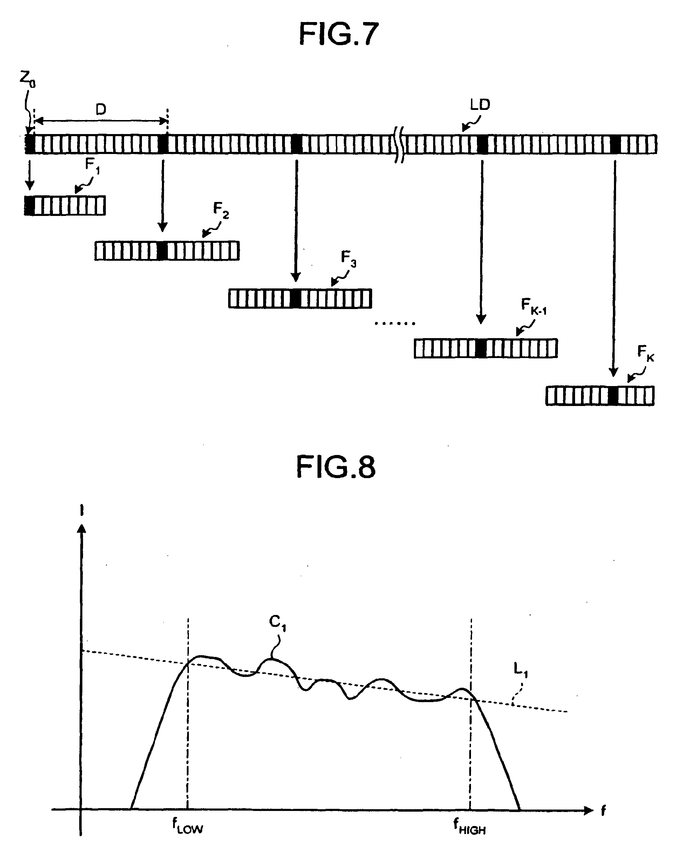

Then, the frequency analyzing unit 41 calculates the frequency spectrum of all of a plurality of data positions set on a single acoustic ray. Regarding that, firstly, the frequency analyzing unit 41 sets an initial value $Z_0$ of a data position Z (equivalent to reception depth) that is representative of a sequence of data groups (FFT data groups) obtained for the purpose of FFT (Step S22). FIG. 7 is a diagram that schematically illustrates data arrangement of a single acoustic ray. In an acoustic ray LD illustrated in FIG. 7, a white rectangle or a black rectangle represents a single set of data. The acoustic ray LD is discretized by time intervals corresponding to the sampling frequency (such as 50 MHz) used during A/D conversion performed by the transmitting-receiving unit 3. In FIG. 7, it is illustrated that the first set of data on the acoustic ray LD is set as the initial value $Z_0$ of the data position Z. Meanwhile, FIG. 7 is only an example, and the position of the initial value $Z_0$ can be set in an arbitrary manner. For example, the data position Z corresponding to the position at the top edge of the area of concern can be set as the initial value $Z_0$.

Then, the frequency analyzing unit 41 obtains the FFT data group at the data position Z (Step S23) and implements the window function, which is stored in the window function storing unit 83, to the FFT data group that has been obtained (Step S24). By implementing the window function to the FFT data group, it becomes possible to avoid discontinuity at the borders in the FFT data group. As a result, artifacts can be prevented from occurring.

Subsequently, the frequency analyzing unit 41 determines whether or not the FFT data group at the data position Z is a normal data group (Step S25). Herein, it is necessary that the number of sets of data in an FFT data group is in power-of-two. In the following explanation, it is assumed that the number of sets of data in the FFT data group is $2^n$ (where n is a positive integer). When an FFT data group is normal, it means that the data position Z is the $2^{n-1}$-th position from the front of the FFT data group. In other words, when an FFT data group is normal, it means that there are $2^{n-1}-1$ (=N) number of sets of data prior to the data position Z, and there are $2^{n-1}$ (=M) number of sets of data subsequent to the data position Z. In the example illustrated in FIG. 7, FFT data groups $F_2$, $F_3$, and $F_{K-1}$ are normal data groups; while FFT data groups $F_1$ and $F_K$ are abnormal data groups. However, in FIG. 7, it is assumed that n=4 (N=7, M=8).

If the determination result of Step S25 indicates that the FFT data group at the data position Z is normal (Yes at Step S25), then the system control proceeds to Step S27 (described later).

If the determination result of Step S25 indicates that the FFT data group at the data position Z is not normal (No at Step S25), then the frequency analyzing unit 41 inserts zero data equivalent to the deficit and generates a normal FFT data group (Step S26). To the FFT data group that is determined to be not normal at Step S25, the window function is implemented prior to the addition of zero data. Hence, even if zero data is inserted, discontinuity in data does not occur. Once the operation at Step S26 is completed, the system control proceeds to Step S27.

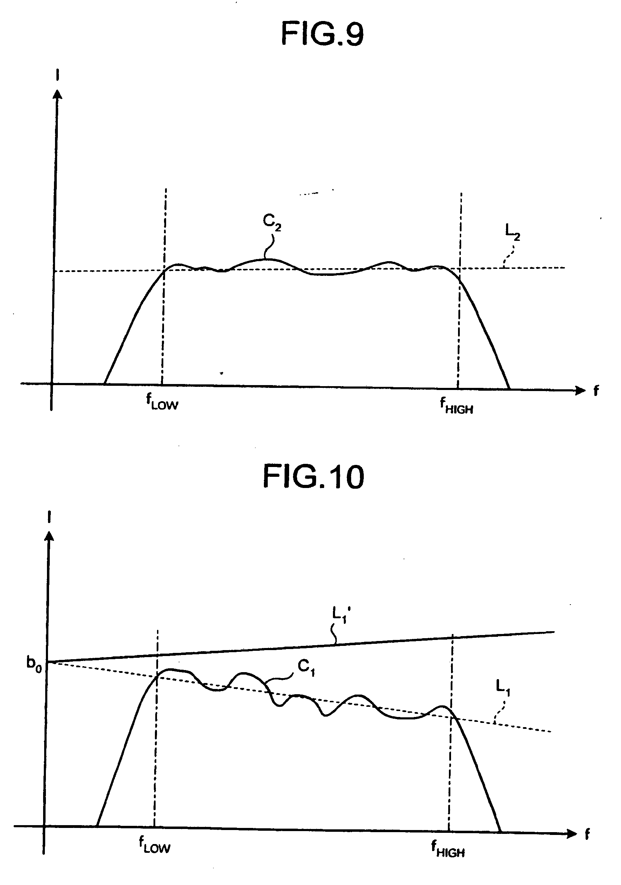

At Step S27, the frequency analyzing unit 41 performs FFT using the FFT data groups and obtains the frequency spectrum (Step S27). FIG. 8 and FIG. 9 are diagrams illustrating examples of the frequency spectrum calculated by the frequency analyzing unit 41. In FIG. 8 and FIG. 9, the horizontal axis f represents the frequency and the vertical axis I represents the intensity. In frequency spectrum curves $C_1$ and $C_2$ illustrated in FIG. 8 and FIG. 9, respectively; a lower limit frequency $f_{LOW}$ and a high limit frequency $f_{HIGH}$ of the frequency spectrum are parameters determined on the basis of the frequency band of the ultrasonic probe 2 and the frequency band of the pulse signals transmitted by the transmitting-receiving unit 3. For example, $f_{LOW}$ is equal to 3 MHz and $f_{HIGH}$ is equal to 10 MHz. Meanwhile, regarding a straight line $L_1$ illustrated in FIG. 8 and a straight line $L_2$ illustrated in FIG. 9, the explanation is given later while explaining the feature data extracting operation. In the first embodiment, curve lines and straight lines are formed of sets of discreet points. The same is the case in other embodiments described later.

Subsequently, the frequency analyzing unit 41 adds a predetermined data step width D to the data position Z, and calculates the data position Z at the FFT data group to be analyzed next (Step S28). Herein, it is desirable that the data step width D is matched with the data step width used at the time when the B-mode image data generating unit 51 generates B-mode image data. However, when the object is to reduce the amount of operations in the frequency analyzing unit 41, it is also possible to set the data step width D to a larger value than the data step width used by the B-mode image data generating unit 51. In FIG. 7, it is illustrated that D=15.

Subsequently, the frequency analyzing unit 41 determines whether or not the data position Z is greater than a final data position $Z_{max}$ (Step S29). Herein, the final data position $Z_{max}$ can be set to the data length of the acoustic ray LD or to the data position corresponding to the lower edge of the area of concern. If the determination result indicates that the data position Z is greater than the final data position $Z_{max}$ (Yes at Step S29), then the frequency analyzing unit 41 increments the acoustic ray number L by 1 (Step S30). On the other hand, if the determination result indicates that the data position Z is equal to or smaller than the final data position $Z_{max}$ (No at Step S29), then the system control returns to Step S23. In this way, with respect to a single acoustic ray LD, the frequency analyzing unit 41 performs FFT for $[\{(Z_{max}-Z_0)/D\}+1]$ (=K) number of FFT data groups. Herein, [X] represents the largest integer not exceeding X.

If the acoustic number L that has been incremented at Step S30 is greater than a final acoustic number $L_{max}$ (Yes at Step S31), then the system control returns to the main routine illustrated in FIG. 4. On the other hand, if the acoustic number L that has been incremented at Step S30 is equal to or smaller than the final acoustic number $L_{max}$ (No at Step S31), then the system control returns to Step S22.

In this way, the frequency analyzing unit 41 performs FFT for K number of times with respect to each of ($L_{max}-L_0+1$) number of acoustic rays. For example, the final acoustic ray number $L_{max}$ can be assigned to the final acoustic ray received by the transmitting-receiving unit 3 or to the acoustic ray corresponding to the border position on any one of the left and right sides of the area of concern. In the following explanation, the total number of times for which the frequency analyzing unit 41 performs FFT with respect to all acoustic rays is ($L_{max}-L_0+1$) $\times$ K and is referred to as "P".

Subsequent to the frequency analyzing operation performed at Step S6 as described above, the approximating unit 421 performs, as an approximation operation, regression analysis of the P number of frequency spectrums calculated by the frequency analyzing unit 41 and extracts the pre-correction feature data (Step S8). More particularly, the approximating unit 421 performs regression analysis and calculates the linear expression for approximation of the frequency spectrums in the frequency band of $f_{LOW}<f<f_{HIGH}$; and then calculates the gradient $a_0$, the intercept $b_0$, and the intensity $c_0$, which characterize the linear expression, as the pre-correction feature data. The straight line $L_1$ illustrated in FIG. 8 and the straight line $L_2$ illustrated in FIG. 9 are regression lines obtained by performing regression analysis of the frequency spectrum curve $C_1$ and the frequency spectrum curve $C_2$, respectively, at Step S8.

Then, the attenuation correcting unit 422 performs attenuation correction of the pre-correction feature data extracted by the approximating unit 421 (Step S9). For example, when the data sampling frequency is 50 MHz, the time interval for data sampling is 20 (nsec). If the velocity of sound is assumed to be 1530 (m/sec), then the spacing among data sampling is equal to 1530 (m/sex)x20 (nsec)/2=0.0153 (mm). If "k" is assumed to be the number of data steps from the first set of data of the acoustic ray LD up to the data position of the FFT data group to be processed, then the data position Z thereof is equal to 0.0153k (mm). The attenuation correcting unit 422 substitutes the value of the data position Z, which is obtained in the manner described above, in the reception depth z specified in Equations (2) to (4) mentioned above, and calculates the gradient a, the intercept b, and the intensity c. FIG. 10 is a diagram illustrating a straight line that is determined from the feature data obtained upon performing attenuation correction of the feature data related to the straight line $L_1$ illustrated in FIG. 8. A straight line $L_1'$ illustrated in FIG. 10 can be expressed as:

$$I=af+b=(a_0+2\alpha Z)f+b_0 \qquad\qquad (5)$$

As is clear from Equation (5), as compared to the straight line $L_1$, the straight line $L_1'$ has a greater gradient with the same intercept value.

Subsequently, based on the feature data extracted by the feature data extracting unit 42 and based on the information on known specimens stored in the known-specimen information storing unit 81, the tissue characterization determining unit 43 determines the tissue characterization of the area of concern of the specimen (Step S10).

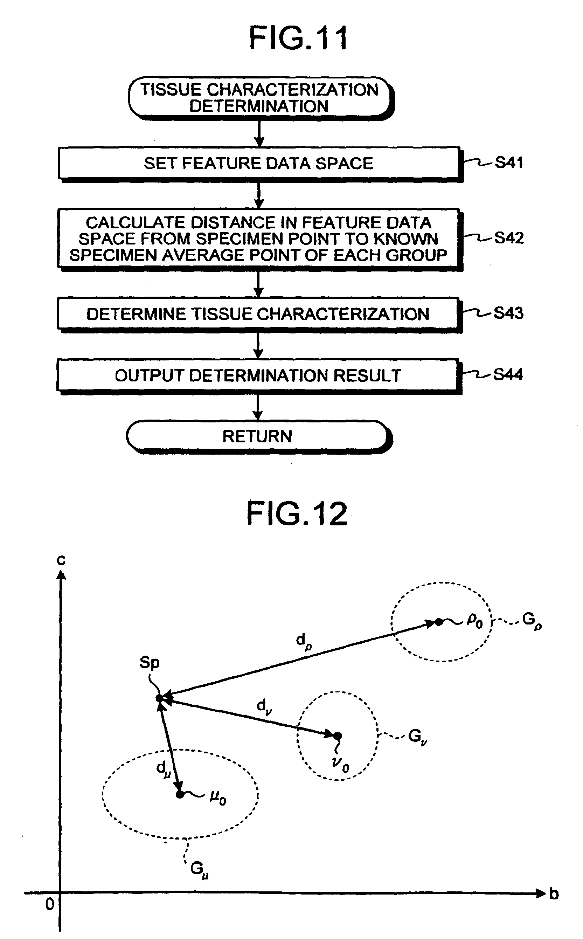

Herein, the operation performed by the tissue characterization determining unit 43 (Step S10) is explained in detail by referring to a flowchart illustrated in FIG. 11. Firstly, the tissue characterization determining unit 43 sets a feature data space that is to be used while determining the tissue characterization (Step S41). In the first embodiment, among the gradient a, the intercept b, and the intensity c that are the three components of feature data; there are two independent parameters. Thus, a two-dimensional space can be set that has any two of the three components of feature data as the components. Alternatively, a one-dimensional space can also be set that has any one of the three components of feature data as the component. At Step S41, it is assumed that the feature data space to be set is determined in advance. However, alternatively, the operator can be allowed to set the desired feature data space using the input unit 6.

FIG. 12 is a diagram illustrating an example of the feature data space set by the tissue characterization determining unit 43. In the feature data space illustrated in FIG. 12, the horizontal axis represents the intercept b and the vertical axis represents the intensity c. In FIG. 12, a point Sp represents the point (hereinafter, referred to as "specimen point Sp") that has the intercept b and the intensity c calculated regarding the target specimen for determination as the coordinates of the feature data space. Moreover, areas $G_\mu$, $G_\nu$, and $G_\rho$ illustrated in FIG. 12 represent groups in which known specimens stored in the known-specimen information storing unit 81 have tissue characterizations of $\mu$, v, and p, respectively. In the example illustrated in FIG. 12, in the feature data space, the three groups $G_\mu$, $G_\nu$, and $G_\rho$ are present in mutually exclusive areas.

In the first embodiment, even while obtaining the feature data of a known specimen; the tissue characterizations are classified and determined with the feature data, which is obtained by performing attenuation correction with respect to the pre-correction feature data of the frequency spectrum obtained by means of frequency analysis, serving as the index. Hence, it becomes possible to make distinction between mutually different tissue characterizations. Particularly, in the first embodiment, the feature data that has been subjected to attenuation correction is used. Therefore, as compared to the case of using the feature data that is extracted without performing attenuation correction, the area of each tissue characterization in the feature data space can be obtained in a more distinctly separated state.

Subsequent to Step S41, the tissue characterization determining unit 43 calculates distances $d_\mu$ $d_v$, and $d_\rho$ from the specimen point Sp to points $\mu_0$, $v_0$, and $\rho_0$, respectively, (hereinafter, these points are referred to as "known specimen average points"); where the points $\mu_0$, $v_0$, and $\rho_0$ have the average of the intercept b and the average of the intensity c of the frequency spectrum of the FFT data group included in the groups $G_\mu$, G $G_v$, and $G_\rho$, respectively, as the coordinates in the feature data space (Step S42). Meanwhile, if the b-axis component and the c-axis component in the feature data space differ in scale by a large extent, it is desirable to appropriately perform weighting so that each distance contributes in a substantially equal manner.

Then, based on the distances calculated at Step S42, the tissue characterization determining unit 43 determines the tissue characterizations of all specimen points including the specimen point Sp (Step S43). For example, in the case illustrated in FIG. 12, since the distance $d_\mu$ is the smallest, the tissue characterization determining unit 43 determines that $_\mu$ is the tissue characterization of the specimen. Meanwhile, if the specimen point Sp is extremely separated from the known specimen average points $\mu_0$, $v_0$, and $\rho_0$; then the determination result of tissue characterizations is low in terms of reliability even if the smallest values of the distances $d_\mu$, $d_v$, and $d_\rho$ are obtained. In that regard, when the distances $d_\mu$, $d_v$, and $d_\rho$ are greater than a predetermined threshold value, the tissue characterization determining unit 43 can be configured to output an error signal. Moreover, when that smallest value of the distances $d_\mu$, $d_v$, and $d_\rho$ is obtained for two or more times; the tissue characterization determining unit 43 can be configured to select all tissue characterizations corresponding to the smallest value or to select only one tissue characterization according to predetermined rules. In the latter case, for example, a method can be implemented in which a high-grade tissue characterization such as cancer is set to have a high priority. Meanwhile, alternatively, when that smallest value of the distances $d_\mu$, d $d_v$, and $d_\rho$ is obtained for two or more times; the tissue characterization determining unit 43 can be configured to output an error signal.

Then, the tissue characterization determining unit 43 outputs the distance calculation result obtained at Step S42 and the determination result obtained at Step S43 (Step S44). That marks the end of the tissue characterization determining operation performed at Step S10.

Subsequent to Step S10 described above, the determination-result-display image data generating unit 52 generates determination-result-display image data by referring to the B-mode image data output by the B-mode image data generating unit 51, the feature data calculated by the feature data extracting unit 42, and the determination result obtained by the tissue characterization determining unit 43 (Step S11).

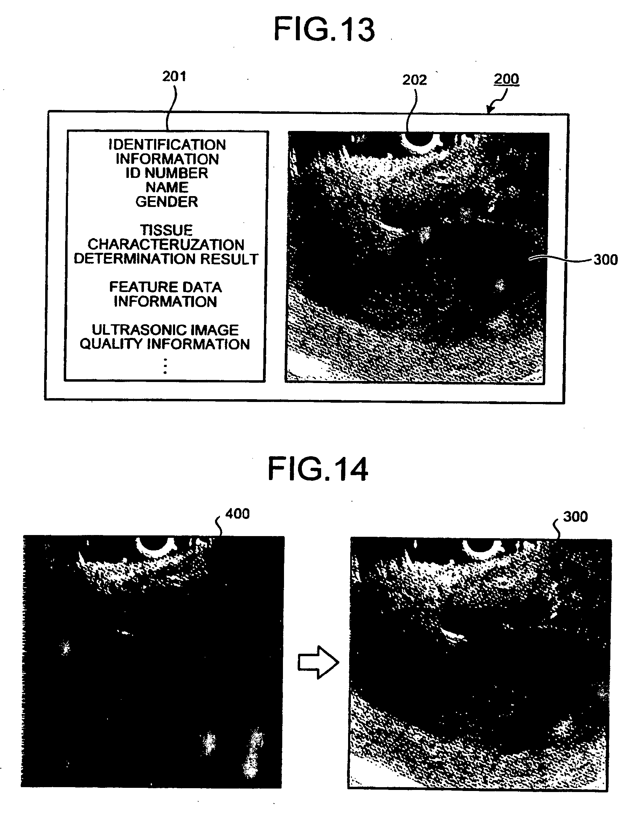

Then, the display unit 7 displays a determination result display image generated by the determination-result-display image data generating unit 52 (Step S12). FIG. 13 is a diagram illustrating a display example of the determination result display image displayed by the display unit 7. A determination result display image 200 illustrated in FIG. 13 includes an information displaying portion 201, which is used for displaying a variety of related information including the tissue characterization determination result, and an image displaying portion 202, which is used for displaying the tissue characterization weighted image in which the tissue characterization is highlighted based on the B-mode image.

In the information displaying portion 201, for example, following information is displayed: identification information (ID number, name, gender) of a specimen; the tissue characterization determination result obtained by the tissue characterization determining unit 43; feature data information used in performing tissue characterization determination; and ultrasonic image quality information such as gain and contrast. Herein, as the feature data information, the display can be performed using the average and the standard deviation of feature data of the frequency spectrums of Q number of FFT data groups present inside the area of concern. More particularly, in the information displaying portion 201, for example, it is possible to display the following information: gradient=$1.5\pm0.3$ (dB/MHz); intercept=$-60\pm2$ (dB); and intensity=$-50\pm1.5$ (dB).

As compared to the B-mode image 100 illustrated in FIG. 5, a tissue characterization weighted image 300 displayed in the image displaying portion 202 is a grayscale image in which the intercept b is evenly assigned among R (red), G (green), and B (blue).

When the display unit 7 displays the determination result display image 200 having the abovementioned configuration, the operator can correctly understand the tissue characterization of the area of concern. However, determination result display images are not limited to the abovementioned configuration. Alternatively, for example, as a determination result display image, it is possible to display side-by-side a tissue characterization weighted image and a B-mode image. With that, the differences in the two images become recognizable on the same screen.

FIG. 14 is a diagram explaining the effect of attenuation correction performed by the ultrasonic diagnosis apparatus 1 according to the first embodiment. An image 400 illustrated in FIG. 14 is a tissue characterization weighted image not subjected to attenuation correction. In the tissue characterization weighted image 400, in the area having a large reception depth (the lower area in FIG. 14), the signal intensity decreases due to the effect of attenuation, thereby making the image darker. In contrast, regarding the tissue characterization weighted image 300 for which attenuation correction is performed, it can be seen that the image has got a uniform brightness throughout the screen.

As described above, according to the first embodiment of the present invention, approximation is performed with respect to a frequency spectrum that has been obtained by analyzing the frequencies of received ultrasonic sound waves. Then, the feature data of a specimen is extracted by performing attenuation correction by which there is a decrease in the contribution of attenuation, which occurs due to the reception depth and the frequency of the ultrasonic sound waves. Based on the extracted feature data of the specimen and based on the feature data of a plurality of known specimens, the tissue characterization of a predetermined area of the specimen is determined. Hence, without having to make use of the strain amount or the degree of elasticity of the body tissues, it becomes possible to make clear distinction between different tissues. As a result, tissue characterizations can be distinguished with accuracy and the measurement result can be enhanced in terms of reliability.

Moreover, according to the first embodiment, since attenuation correction is performed with respect to the extracted feature data, it becomes possible to eliminate the effect of attenuation that occurs during the propagation of ultrasonic sound waves. That makes it possible to determine tissue characterizations with a higher degree of accuracy.

Meanwhile, in the first embodiment, the tissue characterization weighted image 300 described above is only an example. Alternatively, for example, it is also possible to display a tissue characterization weighted image in the form of a color image by assigning R (red), G (green), and B (blue) serving as visual information to each of the gradient a, the intercept, and the intensity c that are the three components of feature data. In this case, in a tissue characterization weighted image, colors are displayed corresponding to tissue characterizations. Hence, based on the color distribution in the image, the operator can understand the tissue characterization of the area of concern. Explained below is a specific example of using a color image.

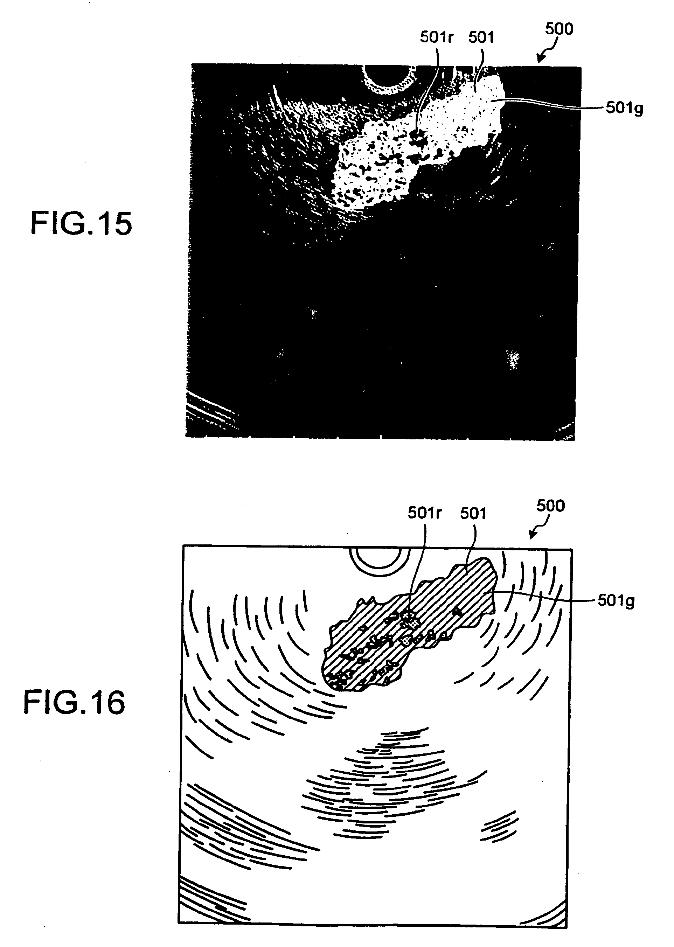

FIG. 15 is a diagram illustrating a display example (first example) of a tissue characterization weighted image in which a color image is used. FIG. 16 is a diagram that schematically illustrates a black-and-white image of the image illustrated in FIG. 15. In a tissue characterization weighted image 500 illustrated in FIG. 15 and FIG. 16, only a specific area 501 is displayed as a color image, and the remaining area is displayed without modification as a B-mode image. The specific area 501 is broadly divided into a greenish area 501g and a reddish area 501r, with the boundary portion between those two areas displayed in a yellowish color (not illustrated in FIG. 16). As illustrated in FIG. 15, it is not the case that each area is made of only a single color. For example, the greenish area 501g is an area including pixels having colors close to the green color. Similarly, the reddish area 501r is an area including pixels having colors close to the red color.

The determination-result-display image data generating unit 52 converts the pixel values of the B-mode image, which corresponds to the specimen point included in a predetermined group in the feature data space, into pixel values colored according to the feature data; and generates the tissue characterization weighted image 500 in which the area 501 is displayed as a color image. With that tissue characterization weighted image 500, it becomes possible to highlight the area of a predetermined tissue characterization as a color image. Hence, the person in charge of diagnosis can easily understand the area in which specific tissues are present. That enables achieving enhancement in the detection rate.

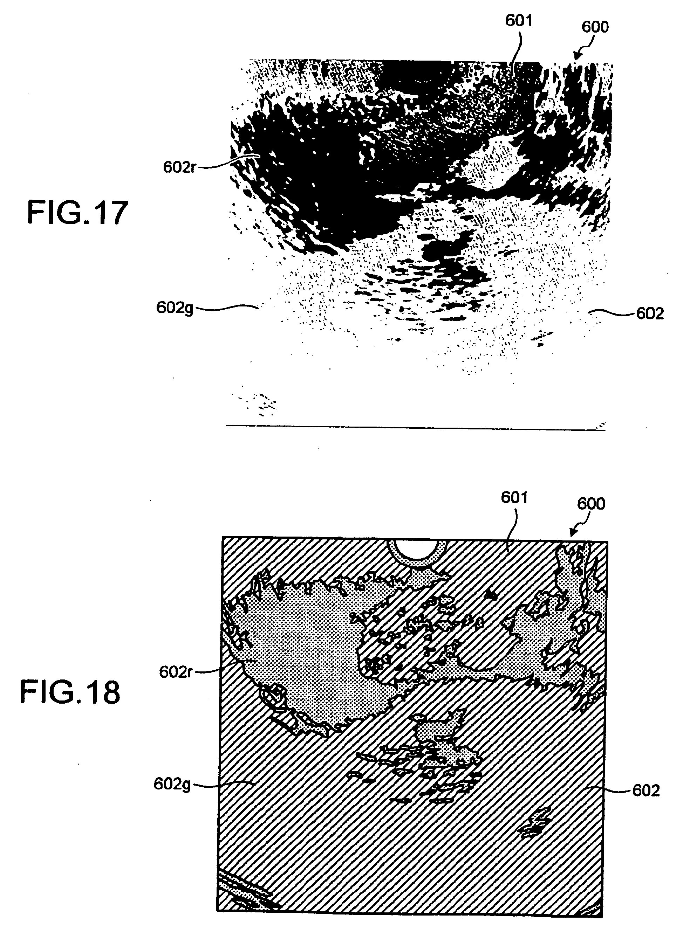

FIG. 17 is a diagram illustrating a display example (second example) of a tissue characterization weighted image in which a color image is used. FIG. 18 is a diagram that schematically illustrates a black-and-white image of the image illustrated in FIG. 17. A tissue characterization weighted image 600 illustrated in FIG. 17 and FIG. 18 is an image generated using the same B-mode image as used in the tissue characterization weighted image 500 illustrated in FIG. 15 and FIG. 16. However, in the tissue characterization weighted image 600; an area 601, which corresponds to the area 501, is displayed without modification as the B-mode image, and a remaining area 602 is displayed as a color image. More particularly, the area 602 is broadly divided into a greenish area 602g and a reddish area 602r, with the boundary portion between those two areas displayed in a yellowish color (not illustrated in FIG. 18). In FIG. 18 too, in an identical manner to FIG. 16, the greenish area 602g is an area including pixels having colors close to the green color; while the reddish area 602r is an area including pixels having colors close to the red color.

The determination-result-display image data generating unit 52 converts the pixel values of the B-mode image, which corresponds to the specimen point included in a predetermined group in the feature data space, into pixel values colored according to the feature data; and generates the tissue characterization weighted image 600 in which the area other than the area 601 is displayed as a color image. With that tissue characterization weighted image 600, it becomes possible to display the area of a predetermined tissue characterization as the B-mode image and to display the remaining area as a color image. Hence, the person in charge of diagnosis can easily understand the area in which specific tissues are present. That enables achieving enhancement in the detection rate. Besides, the internal structure of those tissues can be correctly understood based on the B-mode image.

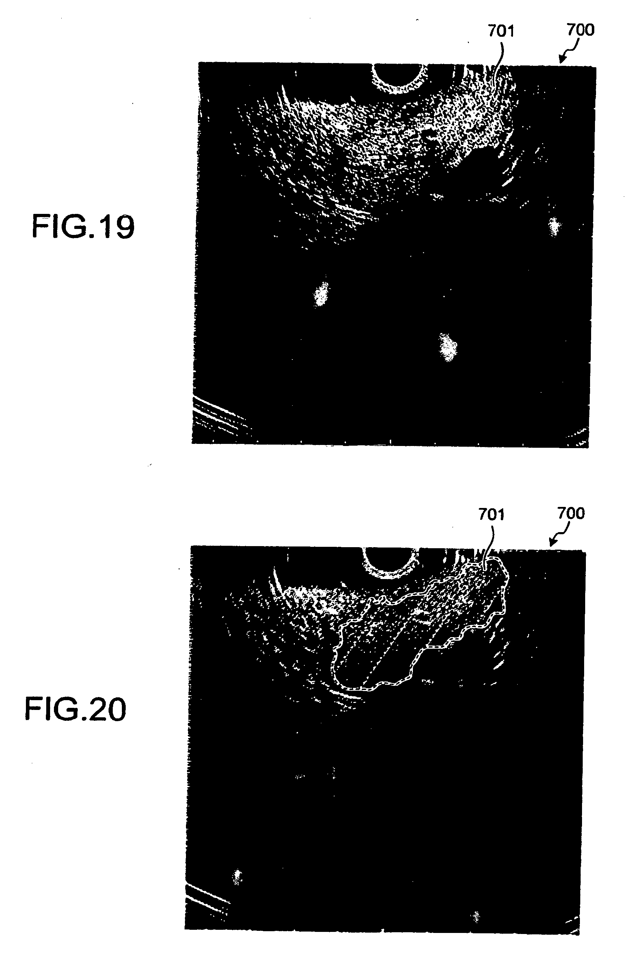

FIG. 19 is a diagram illustrating a display example (third example) of a tissue characterization weighted image in which a color image is used. FIG. 20 is a diagram that schematically illustrates a black-and-white image of the image illustrated in FIG. 19. A tissue characterization weighted image 700 illustrated in FIG. 19 and FIG. 20 is an image generated using the same B-mode image as used in the tissue characterization weighted image 500 illustrated in FIG. 15 and FIG. 16. However, in the tissue characterization weighted image 700; it is not only that an area 701, which corresponds to the area 501, is displayed a color image but also that the display enables to understand the internal structure of the area 701. The determination-result-display image data generating unit 52 performs an operation in which the pixels corresponding to the specimen point included in a predetermined group in the feature data space have new pixel values obtained by weighting the pixel values of the B-mode image and the pixel values of the color image that are determined depending on the tissue characterization, and by taking an average of the weighted pixels values; and generates the tissue characterization weighted image 700 that has the area 701 in which the B-mode image and the color image are displayed in a superposed manner. With that tissue characterization weighted image 700, it becomes possible to display only the area 701 having a predetermined tissue characterization as a color image. Hence, the person in charge of diagnosis can easily understand the area in which specific tissues are present. That enables achieving enhancement in the detection rate. Besides, the internal structure of those tissues can be correctly understood by referring to the information of the B-mode image.

Meanwhile, instead of forming the color space with the RGB color system; it is also possible to form the color space with variables such as cyan, magenta, and yellow of a complementary color system, and to assign feature data to each variable.

(Second embodiment)

In a second embodiment of the present invention, the feature data extracting operation performed by a feature data extracting unit is different than the first embodiment. The configuration of an ultrasonic diagnosis apparatus according to the second embodiment is same as the configuration of the ultrasonic diagnosis apparatus 1 according to the first embodiment. Thus, in the following explanation, the constituent elements identical to those in the ultrasonic diagnosis apparatus 1 are referred to by the same reference numerals.

During the feature data extracting operation according to the second embodiment, firstly, the attenuation correcting unit 422 performs attenuation correction with respect to the frequency spectrum calculated by the frequency analyzing unit 41. Then, the approximating unit 421 performs approximation with respect to the frequency spectrum that has been subjected to attenuation correction by the attenuation correcting unit 422, and extracts the feature data of the frequency spectrum.

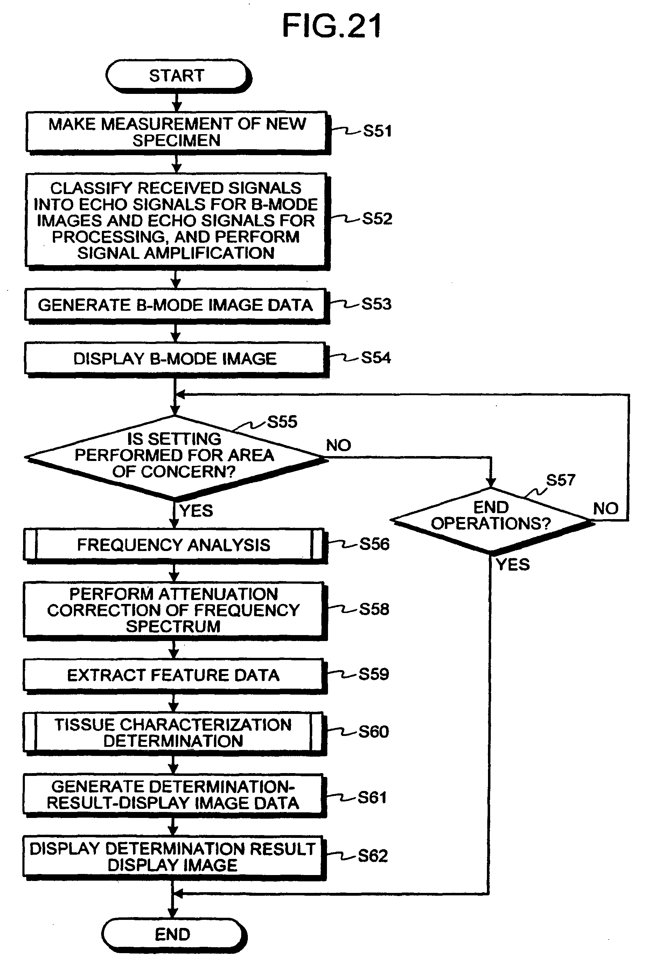

FIG. 21 is a flowchart explaining an overview of the operations performed by the ultrasonic diagnosis apparatus 1 according to the second embodiment. With reference to FIG. 21, the operations performed at Step S51 to Step S57 are respectively identical to the operations performed at Step S1 to Step S7 illustrated in FIG. 4.

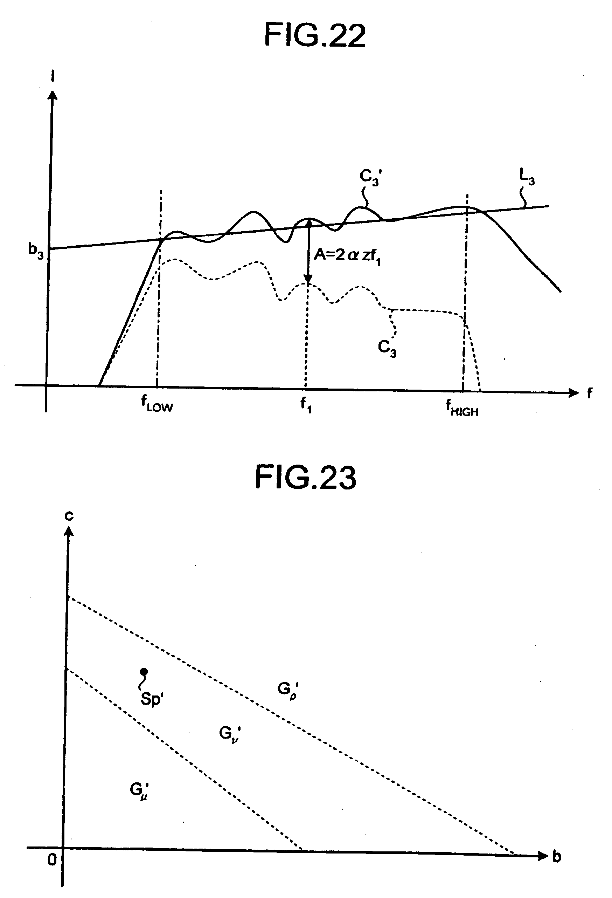

At Step S58, the attenuation correcting unit 422 performs attenuation correction with respect to all frequency spectrums that are calculated by the frequency analyzing unit 41 by means of FFT (Step S58). FIG. 22 is a diagram that schematically illustrates an overview of the operation performed at Step S58. As illustrated in FIG. 22, with respect to a frequency spectrum curve $C_3$, the attenuation correcting unit 422 performs correction in the form of adding the attenuation amount A given in Equation (1) to the intensity I for all frequencies f, and obtains a new frequency spectrum curve $C_3$'. As a result, it becomes possible to obtain a frequency spectrum in which the contribution of attenuation occurring due to the propagation of ultrasonic sound waves is reduced.

Subsequently, the approximating unit 421 performs regression analysis of all frequency spectrums that are subjected to attenuation correction by the attenuation correcting unit 422, and extracts the feature data of the frequency spectrums (Step S59). More particularly, the approximating unit 421 performs regression analysis and calculates the gradient a, the intercept b, and the intensity c at the central frequency $f_{MID}$, which characterize the linear expression. A straight line $L_3$ illustrated in FIG. 22 is a regression line (intercept $b_3$) obtained by performing the feature data extracting operation on the frequency spectrum curve $C_3$ at Step S59.

The operations performed at Step S60 to Step S62 are respectively identical to the operations performed at Step S10 to Step S12 illustrated in FIG. 4.

As described above, according to the second embodiment of the present invention, with respect to a frequency spectrum that has been obtained by analyzing the frequencies of received ultrasonic sound waves, attenuation correction is performed so that there is a decrease in the contribution of attenuation, which occurs due to the reception depth and the frequency of ultrasonic sound waves. Then, the feature data of a specimen is extracted by performing approximation. Subsequently, based on the extracted feature data of the specimen and based on the feature data of a plurality of known specimens, the tissue characterization of a predetermined area of the specimen is determined. Hence, without having to make use of the strain amount or the degree of elasticity of the body tissues, it becomes possible to make clear distinction between different tissues. As a result, tissue characterizations can be distinguished with accuracy and the measurement result can be enhanced in terms of reliability.

Moreover, according to the second embodiment, since attenuation correction is performed with respect to the frequency spectrum, it becomes possible to eliminate the effect of attenuation that occurs during the propagation of ultrasonic sound waves. That makes it possible to determine tissue characterizations with a higher degree of accuracy.

Third embodiment

In a third embodiment of the present invention, the tissue characterization determining operation performed by a tissue characterization determining unit is different than the first embodiment. The configuration of an ultrasonic diagnosis apparatus according to the third embodiment is same as the configuration of the ultrasonic diagnosis apparatus 1 according to the first embodiment. Thus, in the following explanation, the constituent elements identical to those in the ultrasonic diagnosis apparatus 1 are referred to by the same reference numerals.

The tissue characterization determining unit 43 forms new populations by adding the feature data (a, b, c) to each of the groups $G_\mu$, $G_\nu$, and $G_\rho$ that constitute the tissue characterizations $\mu_0$, $\nu_0$, and $\rho_0$, respectively, (see FIG. 12). Then, for each set of feature data, the tissue characterization determining unit 43 obtains the standard deviation of the data constituting each tissue characterization.

Subsequently, the tissue characterization determining unit 43 calculates the difference between the standard deviation of each set of feature data of the groups $G_\mu$, $G_\nu$, and $G_\rho$ in the original populations formed of only known specimens and the standard deviation of each set of feature data of the groups $G_\mu$, $G_\nu$, and $G_\rho$ in the new population obtained by adding the new specimen to each group (hereinafter, the difference is simply referred to as "standard deviation difference"); and determines that the tissue characterization corresponding to the group including the feature data having the smallest standard deviation difference is the tissue characterization of the specimen.

Herein, alternatively, the tissue characterization determining unit 43 can also calculate the standard deviation difference only with respect to the standard deviations of those sets of feature data which are selected in advance from a plurality of sets of feature data. In this case, the selection of sets of feature data can either be arbitrarily done by the operator or be automatically done in the ultrasonic diagnosis apparatus 1.

Alternatively, the tissue characterization determining unit 43 can also calculate, for each group, values by adding an appropriate weight to the standard deviation differences of all sets of feature data; and then determine that the tissue characterization corresponding to the group having the smallest calculated value is the tissue characterization of the specimen. In this case, for example, if the gradient a, the intercept b, and the intensity c represent feature data; then the tissue characterization determining unit 43 makes use of weights $w_a$, $w_b$. and $w_c$, respectively, and calculates "$w_a$•(standard deviation difference of a)+$w_b$•(standard deviation difference of b)+$w_c$•(standard deviation difference of c)". Then, based on the calculated value, the tissue characterization determining unit 43 determines the tissue characterization of the specimen. Herein, the weights $w_a$, $w_b$, and $w_c$ can either be arbitrarily set by the operator or be automatically set in the ultrasonic diagnosis apparatus 1.

Still alternatively, the tissue characterization determining unit 43 can also calculate, for each group, the root square of the value obtained by adding an appropriate weight to the squares of the standard deviation differences of all sets of feature data; and then determine that the tissue characterization corresponding to the group having the smallest square root is the tissue characterization of the specimen. In this case, for example, if the gradient a, the intercept b, and the intensity c represent feature data; then the tissue characterization determining unit 43 makes use of weights $w'_a$, $w'_b$, and $w'_c$, respectively, and calculates "$\{w'_a$•(standard deviation difference of a)$^2$+$w'_b$•(standard deviation difference of b)$^2$+$w'_c$•(standard deviation difference of c)$^2\}^{1/2}$". Then, based on the calculated value, the tissue characterization determining unit 43 determines the tissue characterization of the specimen. Herein too, the weights $w'_a$, $w'_b$, and $w'_c$ can either be arbitrarily set by the operator or be automatically set in the ultrasonic diagnosis apparatus 1.

As described above, according to the third embodiment, in an identical manner to the first embodiment, tissue characterizations can be distinguished with accuracy and the measurement result can be enhanced in terms of reliability. Moreover, it becomes possible to eliminate the effect of attenuation that occurs during the propagation of ultrasonic sound waves. That makes it possible to determine tissue characterizations with a higher degree of accuracy.

In the third embodiment, it is explained that the tissue characterization determining unit 43 determines tissue characterizations based on the changes in the standard deviation difference of each set of feature data between the original population and the new population that is obtained by adding a new specimen to the original population. However, that is only an example. Alternatively, for example, the tissue characterization determining unit 43 can determine tissue characterizations based on the changes in the average of each set of feature data between the original population and the new population that is obtained by adding a new specimen to the original population.

(Fourth embodiment)

In a fourth embodiment of the present invention, the tissue characterization determining operation performed by a tissue characterization determining unit is different than the first embodiment. The configuration of an ultrasonic diagnosis apparatus according to the fourth embodiment is same as the configuration of the ultrasonic diagnosis apparatus 1 according to the first embodiment. Thus, in the following explanation, the constituent elements identical to those in the ultrasonic diagnosis apparatus 1 are referred to by the same reference numerals.

The tissue characterization determining unit 43 calculates the probability belonging to each tissue characterization by referring to the distance between the specimen point in the feature data space and the average point of known specimens. More particularly, in the case of the feature data space (b, c) illustrated in FIG. 12, the distances $d_\mu$, $d_\nu$, and $d_\rho$ from the specimen point Sp to the points $\mu_0$, $\nu_0$, and $\rho_0$, respectively, are used to calculate the probability belonging to each tissue characterization. The setting is so done that smaller the distance, greater is the probability belonging to each known specimen. For example, with $\lambda=100(\alpha^{-1}+\beta^{-1}+\gamma^{-1})$ (%), it can be defined that the probability belonging to a tissue characterization A is $\lambda/\alpha$ (%), the probability belonging to a tissue characterization B is $\lambda/\beta$ (%), and the probability belonging to a tissue characterization C is $\lambda/\gamma$ (%).

In the fourth embodiment, when the display unit 7 displays a determination result display image, the probability belonging to each tissue characterization is displayed in the information displaying portion. For example, when the display unit 7 displays the determination result display image 200, the following determination result is displayed in the information displaying portion 201: "probability of tissue characterization $\mu$=60%, probability of tissue characterization $\nu$=5%, and probability of tissue characterization p=35%".

As described above, according to the fourth embodiment, in an identical manner to the first embodiment, tissue characterizations can be distinguished with accuracy and the measurement result can be enhanced in terms of reliability. Moreover, it becomes possible to eliminate the effect of attenuation that occurs during the propagation of ultrasonic sound waves. That makes it possible to determine tissue characterizations with a higher degree of accuracy.

(Fifth embodiment)

In a fifth embodiment of the present invention, the tissue characterization determining operation performed by a tissue characterization determining unit is different than the first embodiment. The configuration of an ultrasonic diagnosis apparatus according to the fifth embodiment is same as the configuration of the ultrasonic diagnosis apparatus 1 according to the first embodiment. Thus, in the following explanation, the constituent elements identical to those in the ultrasonic diagnosis apparatus 1 are referred to by the same reference numerals.

FIG. 23 is a diagram explaining the overview of the tissue characterization determining operation performed by the tissue characterization determining unit 43 according to the fifth embodiment. In the feature data space illustrated in FIG. 23, the horizontal axis represents the post-attenuation-correction intercept b and the vertical axis represents the post-attenuation-correction intensity c. The feature data space is grouped into areas depending on the tissue characterizations. The tissue characterization determining unit 43 determines the tissue characterization according to the position of the specimen. In FIG. 23, it is illustrated that a point Sp' lies in a group $G_\nu$' (in which $\nu$ is the tissue characterization). In this case, the tissue characterization determining unit 43 determines that $\nu$ is the tissue characterization of the area of concern.

As described above, according to the fifth embodiment, in an identical manner to the first embodiment, tissue characterizations can be distinguished with accuracy and the measurement result can be enhanced in terms of reliability. Moreover, it becomes possible to eliminate the effect of attenuation that occurs during the propagation of ultrasonic sound waves. That makes it possible to determine tissue characterizations with a higher degree of accuracy.

Although the invention has been described with respect to the first to fifth embodiments for a complete and clear disclosure, the appended claims are not to be thus limited but are to be construed as embodying all modifications and alternative constructions that may occur to one skilled in the art that fairly fall within the basic teaching herein set forth.

Reference Signs List

| | |
|---|---|
| 1 | ULTRASONIC DIAGNOSIS APPARATUS |
| 2 | ULTRASONIC PROBE |
| 3 | TRANSMITTING-RECEIVING UNIT |
| 4 | PROCESSING UNIT |
| 5 | IMAGE PROCESSING UNIT |
| 6 | INPUT UNIT |
| 7 | DISPLAY UNIT |

| 8 | MEMORY UNIT |
| 9 | CONTROL UNIT |
| 21 | SIGNAL CONVERTING UNIT |
| 31 | SIGNAL AMPLIFYING UNIT |
| 41 | FREQUENCY ANALYZING UNIT |
| 42 | FEATURE DATA EXTRACTING UNIT |
| 43 | TISSUE CHARACTERIZATION DETERMINING UNIT |
| 51 | B-MODE IMAGE DATA GENERATING UNIT |
| 52 | DETERMINATION-RESULT-DISPLAY IMAGE DATA GENERATING UNIT |
| 81 | KNOWN-SPECIMEN INFORMATION STORING UNIT |
| 82 | GAIN INFORMATION STORING UNIT |
| 83 | WINDOW FUNCTION STORING UNIT |
| 84 | CORRECTION INFORMATION STORING UNIT |
| 100 | B-MODE IMAGE |
| 200 | DETERMINATION RESULT DISPLAY UNIT |
| 201 | INFORMATION DISPLAYING PORTION |
| 202 | IMAGE DISPLAYING PORTION |
| 300, 400, 500, 600, 700 | TISSUE CHARACTERIZATION WEIGHTED IMAGE |
| 421 | APPROXIMATING UNIT |
| 422 | ATTENUATION CORRECTING UNIT |

**Claims**

1. An ultrasonic diagnosis apparatus (1) that transmits ultrasonic sound waves to a specimen and receives ultrasonic sound waves reflected from the specimen, and that determines tissue characterization of the specimen based on the received ultrasonic sound waves, the ultrasonic diagnosis apparatus (1) comprising:

   a frequency analyzing unit (41) that analyzes frequencies of the received ultrasonic sound waves and calculates a frequency spectrum;
   a feature data extracting unit (42) that performs attenuation correction and approximation with respect to the frequency spectrum calculated by the frequency analyzing unit (41) so that there is a decrease in the contribution of attenuation, which occurs due to the reception depth and the frequency of ultrasonic sound waves being propagated, and extracts feature data of the specimen;
   a memory unit (8) that is used to store feature data of frequency spectrums, each being extracted based on ultrasonic sound waves reflected from one of a plurality of known specimens, and tissue characterizations of the known specimens in a corresponding manner; and
   a tissue characterization determining unit (43) that determines tissue characterization of a predetermined area of the specimen by referring to feature data, which is stored by the memory unit (8) in a corresponding manner to the tissue characterizations of the plurality of known specimens, and by referring to the feature data extracted by the feature data extracting unit (42),

**characterized in that**

the feature data extracting unit (42) includeseither

(i) an approximating unit (421) that performs the approximation with respect to the frequency spectrum calculated by the frequency analyzing unit (41) and extracts pre-correction feature data as feature data prior to performing the attenuation correction, and an attenuation correcting unit (422) that performs the attenuation correction with respect to the pre-correction feature data extracted by the approximating unit (421), and extracts feature data of the frequency spectrum; or

(ii) an attenuation correcting unit (422) that performs the attenuation correction with respect to the frequency spectrum, and an approximating unit (421) that performs the approximation with respect to the frequency spectrum corrected by the attenuation correcting unit (422) and extracts feature data of the frequency spectrum.

2. The ultrasonic diagnosis apparatus (1) according to claim 1, wherein, greater the reception depth of ultrasonic sound waves, greater is the extent of correction performed by the attenuation correcting unit (422).

3. The ultrasonic diagnosis apparatus (1) according to claim 2, wherein the approximating unit (421) performs polynomial approximation with respect to the frequency spectrum by means of regression analysis.

4. The ultrasonic diagnosis apparatus (1) according to claim 3, wherein the approximating unit (421) performs linear approximation with respect to the frequency spectrum and extracts a plurality of sets of feature data that include at least two components from among a gradient of the linear expression, an intercept of the linear expression, and an intensity that is determined using the gradient, the intercept, and a specific frequency included in the frequency band of the frequency spectrum.

5. The ultrasonic diagnosis apparatus (1) according to claim 4, wherein the attenuation correcting unit (422) performs correction with respect to at least the gradient and the intensity.

6. The ultrasonic diagnosis apparatus (1) according to claim 4 or 5, wherein
the memory unit (8) stores therein the average of each set of feature data present in groups classified on the basis of tissue characterizations of the plurality of known specimens, and
the tissue characterization determining unit (43) sets a feature data space, which has at least one of the sets of feature data as component, and determines tissue characterization of the specimen based on the distance in the feature data space from a specimen point, for which coordinates in the feature data space indicate sets of feature data serving as components of the feature data space from among the sets of feature data of the frequency spectrum of the specimen, to a known specimen average point, for which coordinates in the feature data space indicate the averages of sets of feature data serving as components of the feature data space from among the sets of feature data in the groups of the known specimens.

7. The ultrasonic diagnosis apparatus (1) according to any one of claims 1 to 5, wherein the tissue characterization determining unit (43) calculates standard deviation of feature data in a population, which is obtained by adding the feature data of the specimen in the groups divided on the basis of tissue characterizations of the plurality of known specimens, and sets tissue characterization of the specimen to tissue characterization corresponding to a group that has the smallest difference between the standard deviation and standard deviation of feature data in the groups.

8. The ultrasonic diagnosis apparatus (1) according to any one of claims 1 to 7, further comprising:

a signal amplifying unit (31) that amplifies reception signals of ultrasonic sound waves received from the specimen; and
a B-mode-display image data generating unit (51) that generates B-mode-display image data by converting the amplitude of the reception signals that have been amplified by the signal amplifying unit (31) into luminance, wherein
with respect to signals to be output to the B-mode-display image data generating unit (51), the signal amplifying unit (31) performs amplification by varying gain depending on reception depth, while with respect to signals to be output to the frequency analyzing unit (41), the signal amplifying unit (31) performs amplification with a constant gain.

9. The ultrasonic diagnosis apparatus (1) according to claim 8, wherein, up to a predetermined reception depth, there is a monotonic increase in the gain with respect to signals to be output to the B-mode-display image data generating unit (51).

10. The ultrasonic diagnosis apparatus (1) according to claim 1, further comprising:

a B-mode-display image data generating unit (51) that generates B-mode-display image data by converting amplitude of reception signals of ultrasonic sound waves into luminance;
a determination-result-display image data generating unit (52) that generates visual information corresponding to the feature data of the specimen and generates determination-result-display image data, which is used in displaying tissue characterization of the specimen, by referring to the visual information that has been generated, the B-mode-display image data generated by the B-mode-display image data generating unit (51), and a determination result of the tissue characterization determining unit (43); and
a display unit (7) that displays an image corresponding to the determination-result-display image data generated by the determination-result-display image data generating unit (52).

11. The ultrasonic diagnosis apparatus (1) according to claim 10, wherein
the determination-result-display image data contains a tissue characterization weighted image in which the tissue characterization determined by the tissue characterization determining unit (43) is highlighted, and
the determination-result-display image data generating unit (52) generates the tissue characterization weighted image by substituting an area determined to be predetermined tissue characterization by the tissue characterization determining unit (43) for a feature data image that has the visual information corresponding to the feature data of the specimen.

12. The ultrasonic diagnosis apparatus (1) according to claim 10, wherein
the determination-result-display image data contains a tissue characterization weighted image in which the tissue characterization determined by the tissue characterization determining unit (43) is highlighted, and
the determination-result-display image data generating unit (52) generates the tissue characterization weighted image by substituting an area other than an area determined by the tissue characterization determining unit (43) to be a predetermined tissue characterization for a feature data image that has the visual information corresponding to the feature data of the specimen.

13. The ultrasonic diagnosis apparatus (1) according to claim 10, wherein
the determination-result-display image data contains a tissue characterization weighted image in which the tissue characterization determined by the tissue characterization determining unit (43) is highlighted, and
the determination-result-display image data generating unit (52) weights mutually-corresponding pixel values in the B-mode display image and in a feature data image that has the visual information corresponding to the feature data of the specimen, and generates the tissue characterization weighted image that has averages of the weighted pixels values as pixel values.

14. The ultrasonic diagnosis apparatus (1) according to any one of claims 10 to 13, wherein the visual information indicates variables constituting a color space.

15. An operation method of an ultrasonic diagnosis apparatus (1) that transmits ultrasonic sound waves to a specimen and receives ultrasonic sound waves reflected from the specimen, and that determines tissue characterization of the specimen based on the received ultrasonic sound waves, the operation method comprising:

a frequency analyzing step including analyzing frequencies of the received ultrasonic sound waves and calculating a frequency spectrum by a frequency analyzing unit (41);
a feature data extracting step including performing attenuation correction and approximation with respect to the frequency spectrum calculated at the frequency analyzing step so that there is a decrease in the contribution of attenuation, which occurs due to the reception depth and the frequency of ultrasonic sound waves being propagated, and extracting feature data of the specimen by a feature data extracting unit (42); and
a tissue characterization determining step including determining tissue characterization of a predetermined area of the specimen by referring to feature data of frequency spectrums, each being extracted based on ultrasonic sound waves reflected from one of a plurality of known specimens, and by referring to the feature data extracted at the feature data extracting step by a tissue characterization determining unit (43),
**characterized in that**

the feature data extracting step includes either

(i) an approximating step including performing the approximation with respect to the frequency spectrum calculated by the frequency analyzing step and extracting pre-correction feature data as feature data prior to performing the attenuation correction, and an attenuation correcting step including performing the attenuation correction with respect to the pre-correction feature data extracted by the approximating step, and extracting feature data of the frequency spectrum; or

(ii) an attenuation correcting step including performing the attenuation correction with respect to the frequency spectrum, and an approximating step including performing the approximation with respect to the frequency spectrum corrected by the attenuation correcting step and extracting feature data of the frequency spectrum.

16. An operation program adapted to operate an ultrasonic diagnosis apparatus (1) that transmits ultrasonic sound waves to a specimen and receives ultrasonic sound waves reflected from the specimen, and that determines tissue characterization of the specimen based on the received ultrasonic sound waves, wherein the operation program instructs the ultrasonic diagnosis apparatus (1) to perform:

a frequency analyzing step including analyzing frequencies of the received ultrasonic sound waves and calculating a frequency spectrum by a frequency analyzing unit (41);

a feature data extracting step including performing attenuation correction and approximation with respect to the frequency spectrum calculated at the frequency analyzing step so that there is a decrease in the contribution of attenuation, which occurs due to the reception depth and the frequency of ultrasonic sound waves being propagated, and extracting feature data of the specimen by a feature data extracting unit (42); and

a tissue characterization determining step including determining tissue characterization of a predetermined area of the specimen by referring to feature data of frequency spectrums, each being extracted based on ultrasonic sound waves reflected from one of a plurality of known specimens, and by referring to the feature data extracted at the feature data extracting step by a tissue characterization determining unit (43),

**characterized in that**

the feature data extracting step includes either

(i) an approximating step including performing the approximation with respect to the frequency spectrum calculated by the frequency analyzing step and extracting pre-correction feature data as feature data prior to performing the attenuation correction, and an attenuation correcting step including performing the attenuation correction with respect to the pre-correction feature data extracted by the approximating step, and extracting feature data of the frequency spectrum; or

(ii) an attenuation correcting step including performing the attenuation correction with respect to the frequency spectrum, and an approximating step including performing the approximation with respect to the frequency spectrum corrected by the attenuation correcting step and extracting feature data of the frequency spectrum.

**Patentansprüche**

1. Ultraschalldiagnosevorrichtung (1), die Ultraschallwellen zu einer Probe sendet und von der Probe reflektierte Ultraschallwellen empfängt, und die eine Gewebecharakterisierung der Probe auf der Basis der empfangenen Ultraschallwellen bestimmt, wobei die Ultraschalldiagnosevorrichtung (1) aufweist:

eine Frequenzanalyseeinheit (41), die Frequenzen der empfangenen Ultraschallwellen analysiert und ein Frequenzspektrum berechnet;

eine Merkmalsdaten-Extraktionseinheit (42), die eine Dämpfungskorrektur und -näherung bezüglich des durch die Frequenzanalyseeinheit (41) berechneten Frequenzspektrums ausführt, so dass der Beitrag der Dämpfung, die aufgrund der Eindringtiefe und der Frequenz der sich ausbreitenden Ultraschallwellen auftritt, abnimmt, und die Merkmalsdaten der Probe extrahiert;

eine Speichereinheit (8), die dazu verwendet wird, Merkmalsdaten von Frequenzspektren, von denen jedes basierend auf Ultraschallwellen, die von einer unter einer Mehrzahl von bekannten Proben reflektierten werden, extrahiert wird, und Gewebecharakterisierungen der bekannten Proben in entsprechender Weise zu speichern; und

eine Gewebecharakterisierung-Bestimmungseinheit (43), die eine Gewebecharakterisierung eines vorbestimmten Bereichs der Probe bestimmt, unter Bezugnahme auf Merkmalsdaten, die von der Speichereinheit (8) in einer entsprechenden Weise zu den Gewebecharakterisierungen der Mehrzahl von bekannten Proben abgespeichert werden, und unter Bezugnahme auf die von der Merkmalsdaten-Extraktionseinheit (42) extrahierten Merkmalsdaten,

**dadurch gekennzeichnet, dass**
die Merkmalsdaten-Extraktionseinheit (42) entweder

(i) eine Näherungseinheit (421), die die Näherung bezüglich des durch die Frequenzanalyseeinheit (41) berechneten Frequenzspektrums ausführt und vor dem Durchführen der Dämpfungskorrektur Vorkorrektur-Merkmalsdaten als Merkmalsdaten extrahiert, und eine Dämpfungskorrektureinheit (422), die die Dämpfungskorrektur bezüglich der von der Näherungseinheit (421) extrahierten Vorkorrektur-Merkmalsdaten ausführt und Merkmalsdaten aus dem Frequenzspektrum extrahiert; oder
(ii) eine Dämpfungskorrektureinheit (422), die die Dämpfungskorrektur bezüglich des Frequenzspektrums ausführt, und eine Näherungseinheit (421), die die Näherung bezüglich des durch die Dämpfungskorrektureinheit (422) korrigierten Frequenzspektrums ausführt und Merkmalsdaten aus dem Frequenzspektrum extrahiert,

aufweist.

2. Ultraschalldiagnosevorrichtung (1) nach Anspruch 1, wobei das Ausmaß der von der Dämpfungskorrektureinheit (422) vorgenommenen Korrektur umso größer ist, je größer die Eindringtiefe der Ultraschallwellen ist.

3. Ultraschalldiagnosevorrichtung (1) nach Anspruch 2, wobei die Näherungseinheit (421) eine polynominale Näherung bezüglich des Frequenzspektrums mittels einer Regressionsanalyse ausführt.

4. Ultraschalldiagnosevorrichtung (1) nach Anspruch 3, wobei die Näherungseinheit (421) eine lineare Näherung bezüglich des Frequenzspektrums ausführt und eine Mehrzahl von Merkmalsdatensätzen extrahiert, die mindestens zwei Komponenten aus dem Gradienten des linearen Ausdrucks, einem Achsenabschnitt des linearen Ausdrucks und einer Intensität aufweisen, die unter Verwendung des Gradienten, des Achsenabschnitts und einer speziellen im Frequenzband des Frequenzspektrums enthaltenen Frequenz bestimmt wird.

5. Ultraschalldiagnosevorrichtung (1) nach Anspruch 4, wobei die Dämpfungskorrektureinheit (422) eine Korrektur mindestens bezüglich des Gradienten und der Intensität ausführt.

6. Ultraschalldiagnosevorrichtung (1) nach Anspruch 4 oder 5, wobei
die Speichereinheit (8) einen Mittelwert von jedem Satz von Merkmalsdaten speichert, die in auf der Basis von Gewebecharakterisierungen der Mehrzahl von bekannten Proben klassifizierten Gruppen vorliegen, und
die Gewebecharakterisierung-Bestimmungseinheit (43) einen Merkmalsdatenraum festlegt, der zumindest einen der Merkmalsdatensätze als Komponente aufweist, und Gewebecharakterisierungen der Proben bestimmt, basierend auf dem Abstand im Merkmalsdatenraum zwischen einem Probenpunkt, für den Koordinaten in dem Merkmalsdatenraum Merkmalsdatensätze angeben, die als Komponenten des Merkmalsdatenraums unter den Merkmalsdatensätzen des Frequenzspektrums der Probe dienen, und einem bekannten Durchschnittsprobenpunkt, für den Koordinaten in dem Merkmalsdatenraum die Durchschnitte der Merkmalsdatensätze angeben, die als Komponenten des Merkmalsdatenraums unter den Merkmalsdatensätzen in den Gruppen bekannter Proben dienen.

7. Ultraschalldiagnosevorrichtung (1) nach einem beliebigen der Ansprüche 1 bis 5, wobei die Gewebecharakterisierung-Bestimmungseinheit (43) eine Standardabweichung von Merkmalsdaten in einer Grundgesamtheit berechnet, die erhalten wird durch Addieren der Merkmalsdaten der Proben in den Gruppen geteilt auf der Basis von Gewebecharakterisierungen der Mehrzahl von bekannten Proben, und eine Gewebecharakterisierung der Probe festlegt auf eine Gewebecharakterisierung, die einer Gruppe, welche die geringste Differenz zwischen der Standardabweichung und der Standardabweichung von Merkmalsdaten in den Gruppen aufweist, entspricht.

8. Ultraschalldiagnosevorrichtung (1) nach einem beliebigen der Ansprüche 1 bis 7, die ferner aufweist:

eine Signalverstärkungseinheit (31), die Ultraschallwellen-Empfangssignale verstärkt, die von der Probe empfangen werden; und
eine B-Mode-Display-Bilddatenerzeugungseinheit (51), die B-Mode-Display-Bilddaten erzeugt, indem sie die Amplitude der von der Signalverstärkungseinheit (31) verstärkten Empfangssignale in Leuchtdichte umwandelt,

EP 2 548 512 B1

wobei

in Bezug auf Signale, die an die B-Mode-Display-Bilddatenerzeugungseinheit (51) auszugeben sind, die Signalverstärkungseinheit (31) die Verstärkung mit einem je nach der Eindringtiefe unterschiedlichem Verstärkungsgrad ausführt, während in Bezug auf Signale, die an die Frequenzanalyseeinheit (41) auszugeben sind, die Signalverstärkungseinheit (31) die Verstärkung mit einem gleichbleibenden Verstärkungsgrad ausführt.

9. Ultraschalldiagnosevorrichtung (1) nach Anspruch 8, wobei in Bezug auf Signale, die an die B-Mode-Display-Bilddatenerzeugungseinheit (51) auszugeben sind, bis zu einer vorbestimmten Eindringtiefe ein gleichbleibender Anstieg im Verstärkungsgrad vorliegt.

10. Ultraschalldiagnosevorrichtung (1) nach Anspruch 1, die ferner aufweist:

eine B-Mode-Display-Bilddatenerzeugungseinheit (51), die B-Mode-Display-Bilddaten erzeugt, indem sie die Amplitude von Ultraschallwellen-Empfangssignalen in Leuchtdichte umwandelt;
eine Bestimmungsergebnis-Display-Bilddatenerzeugungseinheit (52), die den Merkmalsdaten der Probe entsprechende optische Informationen erzeugt und die zum Anzeigen einer Gewebecharakterisierung der Probe verwendete Bestimmungsergebnis-Display-Bilddaten erzeugt, unter Bezugnahme auf die erzeugten optischen Informationen, die von der B-Mode-Display-Bilddatenerzeugungseinheit (51) erzeugten B-Mode-Display-Bilddaten, und ein Bestimmungsergebnis der Gewebecharakterisierung-Bestimmungseinheit (43); und
eine Anzeigeeinheit (7), die ein Bild anzeigt, das den von der Bestimmungsergebnis-Display-Bilddatenerzeugungseinheit (52) erzeugten Bestimmungsergebnis-Display-Bilddaten entspricht.

11. Ultraschalldiagnosevorrichtung (1) nach Anspruch 10, wobei
die Bestimmungsergebnis-Display-Bilddaten ein Gewebecharakterisierunggewichtetes Bild enthalten, in dem die von der Gewebecharakterisierung-Bestimmungseinheit (43) bestimmte Gewebecharakterisierung hervorgehoben ist, und
die Bestimmungsergebnis-Display-Bilddatenerzeugungseinheit (52) das Gewebecharakterisierung-gewichtete Bild erzeugt, indem sie einen von der Gewebecharakterisierung-Bestimmungseinheit (43) als eine vorbestimmte Gewebecharakterisierung bestimmten Bereich durch ein Merkmalsdatenbild ersetzt, das die den Merkmalsdaten der Probe entsprechenden optischen Informationen aufweist.

12. Ultraschalldiagnosevorrichtung (1) nach Anspruch 10, wobei
die Bestimmungsergebnis-Display-Bilddaten ein Gewebecharakterisierunggewichtetes Bild enthalten, in dem die von der Gewebecharakterisierung-Bestimmungseinheit (43) bestimmte Gewebecharakterisierung hervorgehoben ist, und
die Bestimmungsergebnis-Display-Bilddatenerzeugungseinheit (52) das Gewebecharakterisierung-gewichtete Bild erzeugt, indem sie einen anderen als einen von der Gewebecharakterisierung-Bestimmungseinheit (43) als eine vorbestimmte Gewebecharakterisierung bestimmten Bereich durch ein Merkmalsdatenbild ersetzt, das die den Merkmalsdaten der Probe entsprechenden optischen Informationen aufweist.

13. Ultraschalldiagnosevorrichtung (1) nach Anspruch 10, wobei
die Bestimmungsergebnis-Display-Bilddaten ein Gewebecharakterisierunggewichtetes Bild enthalten, in dem die von der Gewebecharakterisierung-Bestimmungseinheit (43) bestimmte Gewebecharakterisierung hervorgehoben ist, und
die Bestimmungsergebnis-Display-Bilddatenerzeugungseinheit (52) sich gegenseitig entsprechende Pixelwerte in dem B-Mode-Displaybild und in einem Merkmalsdatenbild, das die den Merkmalsdaten der Probe entsprechenden optischen Informationen aufweist, gewichtet, und das Gewebecharakterisierung-gewichtete Bild erzeugt, das Durchschnittswerte der gewichteten Pixelwerte as Pixelwerte enthält.

14. Ultraschalldiagnosevorrichtung (1) nach einem beliebigen der Ansprüche 10 bis 13, wobei die optischen Informationen einen Farbraum darstellende Größen angeben.

15. Verfahren zum Betreiben einer Ultraschalldiagnosevorrichtung (1), die Ultraschallwellen zu einer Probe sendet und von der Probe reflektierte Ultraschallwellen empfängt, und die eine Gewebecharakterisierung der Probe auf der Basis der empfangenen Ultraschallwellen bestimmt, wobei das Betriebsverfahren aufweist:

einen Frequenzanalyseschritt, der ein Analysieren von Frequenzen der empfangenen Ultraschallwellen und ein Berechnen eines Frequenzspektrums durch eine Frequenzanalyseeinheit (41) beinhaltet;

21

einen Merkmalsdaten-Extraktionsschritt, beinhaltend das Ausführen einer Dämpfungskorrektur und -näherung bezüglich des im Frequenzanalyseschritt berechneten Frequenzspektrums, so dass der Beitrag der Dämpfung, die aufgrund der Eindringtiefe und der Frequenz von sich ausbreitenden Ultraschallwellen auftritt, abnimmt, und das Extrahieren von Merkmalsdaten der Probe durch eine Merkmalsdaten-Extraktionseinheit (42); und einen Gewebecharakterisierung-Bestimmungsschritt, beinhaltend die Bestimmung einer Gewebecharakterisierung eines vorbestimmten Bereichs der Probe durch Bezugnahme auf Merkmalsdaten von Frequenzspektren, von denen jedes basierend auf Ultraschallwellen, die von einer unter einer Mehrzahl von bekannten Proben reflektierten werden, extrahiert wird, und durch Bezugnahme auf die im Merkmalsdaten-Extraktionsschritt durch eine Gewebecharakterisierung-Bestimmungseinheit (43) extrahierten Merkmalsdaten,
**dadurch gekennzeichnet, dass**
der Merkmalsdaten-Extraktionsschritt entweder

(i) einen Näherungsschritt, beinhaltend das Ausführen der Näherung bezüglich des durch den Frequenzanalyseschritt berechneten Frequenzspektrums und das Extrahieren von Vorkorrektur-Merkmalsdaten als Merkmalsdaten vor dem Durchführen der Dämpfungskorrektur, und eine Dämpfungskorrekturschritt, beinhaltend das Ausführen der Dämpfungskorrektur bezüglich der durch den Näherungsschritt extrahierten Vorkorrektur-Merkmalsdaten und das Extrahieren von Merkmalsdaten aus dem Frequenzspektrum; oder
(ii) einen Dämpfungskorrekturschritt beinhaltend, das Ausführen der Dämpfungskorrektur bezüglich des Frequenzspektrums, und einen Näherungsschritt beinhaltend das Ausführen der Näherung bezüglich des durch den Dämpfungskorrekturschritt korrigierten Frequenzspektrums und das Extrahieren von Merkmalsdaten aus dem Frequenzspektrum,

beinhaltet.

16. Betriebsprogramm, das zum Betreiben einer Ultraschalldiagnosevorrichtung (1) ausgelegt ist, die Ultraschallwellen zu einer Probe sendet und von der Probe reflektierte Ultraschallwellen empfängt, und die eine Gewebecharakterisierung der Probe auf der Basis der empfangenen Ultraschallwellen bestimmt, wobei das Betriebsprogramm die Ultraschalldiagnosevorrichtung (1) anweist die folgenden Schritte auszuführen:

einen Frequenzanalyseschritt, der ein Analysieren von Frequenzen der empfangenen Ultraschallwellen und ein Berechnen eines Frequenzspektrums durch eine Frequenzanalyseeinheit (41) beinhaltet;
einen Merkmalsdaten-Extraktionsschritt, beinhaltend das Ausführen einer Dämpfungskorrektur und -näherung bezüglich des im Frequenzanalyseschritt berechneten Frequenzspektrums, so dass der Beitrag der Dämpfung, die aufgrund der Eindringtiefe und der Frequenz von sich ausbreitenden Ultraschallwellen auftritt, abnimmt, und das Extrahieren von Merkmalsdaten der Probe durch eine Merkmalsdaten-Extraktionseinheit (42); und einen Gewebecharakterisierung-Bestimmungsschritt, beinhaltend die Bestimmung einer Gewebecharakterisierung eines vorbestimmten Bereichs der Probe durch Bezugnahme auf Merkmalsdaten von Frequenzspektren, von denen jedes basierend auf Ultraschallwellen, die von einer unter einer Mehrzahl von bekannten Proben reflektierten werden, extrahiert wird, und durch Bezugnahme auf die im Merkmalsdaten-Extraktionsschritt durch eine Gewebecharakterisierung-Bestimmungseinheit (43) extrahierten Merkmalsdaten,
**dadurch gekennzeichnet, dass**
der Merkmalsdaten-Extraktionsschritt entweder

(i) einen Näherungsschritt, beinhaltend das Ausführen der Näherung bezüglich des durch den Frequenzanalyseschritt berechneten Frequenzspektrums und das Extrahieren von Vorkorrektur-Merkmalsdaten als Merkmalsdaten vor dem Durchführen der Dämpfungskorrektur, und eine Dämpfungskorrekturschritt, beinhaltend das Ausführen der Dämpfungskorrektur bezüglich der durch den Näherungsschritt extrahierten Vorkorrektur-Merkmalsdaten und das Extrahieren von Merkmalsdaten aus dem Frequenzspektrum; oder
(ii) einen Dämpfungskorrekturschritt beinhaltend, das Ausführen der Dämpfungskorrektur bezüglich des Frequenzspektrums, und einen Näherungsschritt beinhaltend das Ausführen der Näherung bezüglich des durch den Dämpfungskorrekturschritt korrigierten Frequenzspektrums und das Extrahieren von Merkmalsdaten aus dem Frequenzspektrum,

beinhaltet.

**Revendications**

1. Appareil de diagnostic par ultrasons (1) qui transmet des ondes sonores à ultrasons vers un spécimen et reçoit les ondes sonores à ultrasons réfléchies du spécimen, et qui détermine une caractérisation tissulaire du spécimen sur la base des ondes sonores à ultrasons reçues, l'appareil de diagnostic par ultrasons (1) comprenant :

   une unité (41) d'analyse de fréquence qui analyse les fréquences des ondes sonores à ultrasons reçues et calcule un spectre de fréquence ;
   une unité (42) d'extraction de données de caractéristiques qui réalise une correction d'atténuation et une approximation par rapport au spectre de fréquence calculé par l'unité (41) d'analyse de fréquence de sorte qu'il existe une diminution dans la contribution d'atténuation, qui se produit en raison de la profondeur de réception et de la fréquence des ondes sonores à ultrasons étant propagées, et extrait des données de caractéristiques du spécimen ;
   une unité de mémoire (8) qui est utilisée pour mémoriser des données de caractéristiques des spectres de fréquence, chacune étant extraite sur la base des ondes sonores à ultrasons réfléchies de l'un parmi une pluralité de spécimens connus, et des caractérisations tissulaires des spécimens connus d'une manière correspondante ; et
   une unité (43) de détermination de caractérisation tissulaire qui détermine une caractérisation tissulaire d'une zone prédéterminée du spécimen en se référant aux données de caractéristiques, qui sont mémorisées par l'unité de mémoire (8) d'une manière qui correspond aux caractérisations tissulaires de la pluralité de spécimens connus, et en se référant aux données de caractéristiques extraites par l'unité (42) d'extraction de données de caractéristiques,
   **caractérisé en ce que**
   l'unité (42) d'extraction de données de caractéristiques comprend soit

   (i) une unité (421) d'approximation qui réalise l'approximation par rapport au spectre de fréquence calculé par l'unité (41) d'analyse de fréquence et extrait des données de caractéristiques de précorrection en tant que données de caractéristiques avant de réaliser la correction d'atténuation, et une unité (422) de correction d'atténuation qui réalise la correction d'atténuation par rapport aux données de caractéristiques de précorrection extraites par l'unité (421) d'approximation, et extrait des données de caractéristiques du spectre de fréquence ; soit
   (ii) une unité (422) de correction d'atténuation qui réalise la correction d'atténuation par rapport au spectre de fréquence, et une unité (421) d'approximation qui réalise l'approximation par rapport au spectre de fréquence corrigé par l'unité (422) de correction d'atténuation et extrait des données de caractéristiques du spectre de fréquence.

2. Appareil de diagnostic par ultrasons (1) selon la revendication 1, dans lequel, plus la profondeur de réception des ondes sonores à ultrasons est importante, plus l'étendue de correction réalisée par l'unité (422) de correction d'atténuation est importante.

3. Appareil de diagnostic par ultrasons (1) selon la revendication 2, dans lequel l'unité (421) d'approximation réalise une approximation polynomiale par rapport au spectre de fréquence au moyen d'une analyse de régression.

4. Appareil de diagnostic par ultrasons (1) selon la revendication 3, dans lequel l'unité (421) d'approximation réalise une approximation linéaire par rapport au spectre de fréquence et extrait une pluralité d'ensembles de données de caractéristiques qui contiennent au moins deux composantes parmi un gradient de l'expression linéaire, un point d'intersection de l'expression linéaire, et une intensité qui est déterminée en utilisant le gradient, le point d'intersection et une fréquence spécifique contenue dans la bande de fréquences du spectre de fréquence.

5. Appareil de diagnostic par ultrasons (1) selon la revendication 4, dans lequel l'unité (422) de correction d'atténuation réalise une correction au moins par rapport au gradient et à l'intensité.

6. Appareil de diagnostic par ultrasons (1) selon la revendication 4 ou 5, dans lequel
   l'unité de mémoire (8) mémorise dans celle-ci la moyenne de chaque ensemble de données de caractéristiques se trouvant dans des groupes classifiés sur la base des caractérisations tissulaires de la pluralité de spécimens connus, et
   l'unité (43) de détermination de caractérisation tissulaire établit un espace de données de caractéristiques, qui comporte au moins l'un parmi les ensembles de données de caractéristiques en tant que composante, et détermine une caractérisation tissulaire du spécimen sur la base de la distance dans l'espace de données de caractéristiques depuis un point de spécimen, pour lequel des coordonnées dans l'espace de données de caractéristiques indiquent des ensembles de données de caractéristiques servant de composante de l'espace de données de caractéristiques parmi les ensembles de données de caractéristiques du spectre de fréquence du spécimen, jusqu'à un point moyen de spécimen connu, pour lequel des coordonnées dans l'espace de données de caractéristiques indiquent les moyennes des ensembles de données de caractéristiques servant de composantes de l'espace de données de caractéristiques parmi les ensembles de données de caractéristiques dans les groupes des spécimens connus.

7.  Appareil de diagnostic par ultrasons (1) selon l'une quelconque des revendications 1 à 5, dans lequel l'unité (43) de détermination de caractérisation tissulaire calcule une déviation standard des données de caractéristiques dans une population, qui est obtenue en additionnant les données de caractéristiques du spécimen dans les groupes divisées sur la base des caractérisations tissulaires de la pluralité de spécimens connus, et établit une caractérisation tissulaire du spécimen à une caractérisation tissulaire correspondant à un groupe qui présente la plus petite différence entre la déviation standard et une déviation standard des données de caractéristiques dans les groupes.

8.  Appareil de diagnostic par ultrasons (1) selon l'une quelconque des revendications 1 à 7, comprenant en outre :

    une unité (31) d'amplification de signal qui amplifie des signaux de réception des ondes sonores à ultrasons reçues depuis le spécimen ; et
    une unité (51) de génération de données d'image d'affichage en mode B qui génère des données d'image d'affichage en mode B en convertissant l'amplitude des signaux de réception qui ont été amplifiés par l'unité (31) d'amplification de signal en luminance, dans lequel
    en ce qui concerne les signaux devant être délivrés en sortie vers l'unité (51) de génération de données d'image d'affichage en mode B, l'unité (31) d'amplification de signal réalise une amplification en variant le gain en fonction de la profondeur de réception, alors qu'en ce qui concerne les signaux devant être délivrés en sortie vers l'unité (41) d'analyse de fréquence, l'unité (31) d'amplification de signal réalise une amplification avec un gain constant.

9.  Appareil de diagnostic par ultrasons (1) selon la revendication 8, dans lequel, jusqu'à une profondeur de réception prédéterminée, il existe une augmentation monotone du gain en ce qui concerne les signaux devant être délivrés en sortie vers l'unité (51) de génération de données d'image d'affichage en mode B.

10. Appareil de diagnostic par ultrasons (1) selon la revendication 1, comprenant en outre :

    une unité (51) de génération de données d'image d'affichage en mode B qui génère des données d'image d'affichage en mode B en convertissant l'amplitude des signaux de réception des ondes sonores à ultrasons en luminance ;
    une unité (52) de génération de données d'image d'affichage de résultat de détermination qui génère des informations visuelles correspondant aux données de caractéristiques du spécimen et génère des données d'image d'affichage de résultat de détermination, qui sont utilisées dans l'affichage de caractérisation tissulaire du spécimen, en se référant aux informations visuelles qui ont été générées, les données d'image d'affichage en mode B générées par l'unité (51) de génération de données d'image d'affichage en mode B, et un résultat de détermination de l'unité (43) de détermination de caractérisation tissulaire ; et
    une unité d'affichage (7) qui affiche une image correspondant aux données d'image d'affichage de résultat de détermination générées par l'unité (52) de génération de données d'image d'affichage de résultat de détermination.

11. Appareil de diagnostic par ultrasons (1) selon la revendication 10, dans lequel
    les données d'image d'affichage de résultat de détermination contiennent une image pondérée de caractérisation tissulaire dans laquelle la caractérisation tissulaire déterminée par l'unité (43) de détermination de caractérisation tissulaire est mise en évidence, et
    l'unité (50) de génération de données d'image d'affichage de résultat de détermination génère l'image pondérée de caractérisation tissulaire en substituant une zone déterminée comme étant une caractérisation tissulaire prédéterminée par l'unité (43) de détermination de caractérisation tissulaire par une image de données de caractéristiques qui comporte les informations visuelles correspondant aux données de caractéristiques du spécimen.

12. Appareil de diagnostic par ultrasons (1) selon la revendication 10, dans lequel
    les données d'image d'affichage de résultat de détermination contiennent une image pondérée de caractérisation tissulaire dans laquelle la caractérisation tissulaire déterminée par l'unité (43) de détermination de caractérisation tissulaire est mise en évidence, et

l'unité (50) de génération de données d'image d'affichage de résultat de détermination génère l'image pondérée de caractérisation tissulaire en substituant une zone autre qu'une zone déterminée par l'unité (43) de détermination de caractérisation tissulaire comme étant une caractérisation tissulaire prédéterminée par une image de données de caractéristiques qui comporte les informations visuelles correspondant aux données de caractéristiques du spécimen.

13. Appareil de diagnostic par ultrasons (1) selon la revendication 10, dans lequel

les données d'image d'affichage de résultat de détermination contiennent une image pondérée de caractérisation tissulaire dans laquelle la caractérisation tissulaire déterminée par l'unité (43) de détermination de caractérisation tissulaire est mise en évidence, et

l'unité (52) de génération de données d'image d'affichage de résultat de détermination pondère des valeurs de pixel correspondant mutuellement dans l'image d'affichage en mode B et dans une image de données de caractéristiques qui comporte l'information visuelle correspondant aux données de caractéristiques du spécimen, et génère l'image pondérée de caractérisation tissulaire qui présente des moyennes des valeurs de pixel pondérées en tant que valeurs de pixel.

14. Appareil de diagnostic par ultrasons (1) selon l'une quelconque des revendications 10 à 13, dans lequel les informations visuelles indiquent des variables constituant un espace de couleurs.

15. Procédé de fonctionnement d'un appareil de diagnostic par ultrasons (1) qui transmet des ondes sonores à ultrasons vers le spécimen et reçoit des ondes sonores à ultrasons réfléchies du spécimen, et qui détermine une caractérisation tissulaire du spécimen sur la base des ondes sonores à ultrasons reçues, le procédé de fonctionnement comprenant :

une étape d'analyse de fréquence incluant l'analyse des fréquences des ondes sonores à ultrasons reçues et le calcul d'un spectre de fréquence par une unité (41) d'analyse de fréquence ;

une étape d'extraction de données de caractéristiques incluant l'exécution d'une correction d'atténuation et d'une approximation par rapport au spectre de fréquence calculé à l'étape d'analyse de fréquence de sorte qu'il existe une diminution de la contribution d'atténuation, qui se produit en raison de la profondeur de réception et de la fréquence des ondes sonores à ultrasons étant propagées, et l'extraction de données de caractéristiques du spécimen par une unité (42) d'extraction de données de caractéristiques ; et

une étape de détermination de caractérisation tissulaire incluant la détermination d'une caractérisation tissulaire d'une zone prédéterminée du spécimen en se référant aux données de caractéristiques des spectres de fréquence, chacune étant extraite sur la base des ondes sonores à ultrasons réfléchies de l'un parmi une pluralité de spécimens connus, et en se référant aux données de caractéristiques extraites à l'étape d'extraction de données de caractéristiques par une unité (43) de détermination de caractérisation tissulaire,

**caractérisé en ce que**

l'étape d'extraction de données de caractéristiques comprend soit

(i) une étape d'approximation incluant l'exécution de l'approximation par rapport au spectre de fréquence calculé par l'étape d'analyse de fréquence et l'extraction de données de caractéristiques de précorrection en tant que données de caractéristiques avant d'exécuter la correction d'atténuation, et une étape de correction d'atténuation incluant l'exécution de la correction d'atténuation par rapport aux données de caractéristiques de précorrection extraites par l'étape d'approximation, et l'extraction de données de caractéristiques du spectre de fréquence ; soit

(ii) une étape de correction d'atténuation incluant l'exécution de la correction d'atténuation par rapport au spectre de fréquence, et une étape d'approximation incluant l'exécution de l'approximation par rapport au spectre de fréquence corrigé par l'étape de correction d'atténuation et l'extraction de données de caractéristiques du spectre de fréquence.

16. Programme de fonctionnement adapté pour actionner un appareil de diagnostic par ultrasons (1) qui transmet des ondes sonores à ultrasons vers un spécimen et reçoit des ondes sonores à ultrasons réfléchies du spécimen, et qui détermine une caractérisation tissulaire du spécimen sur la base des ondes sonores à ultrasons reçues, dans lequel le programme de fonctionnement délivre une instruction à l'appareil de diagnostic par ultrasons (1) pour exécuter :

une étape d'analyse de fréquence incluant l'analyse des fréquences des ondes sonores à ultrasons reçues et le calcul d'un spectre de fréquence par une unité (41) d'analyse de fréquence ;

une étape d'extraction de données de caractéristiques incluant l'exécution d'une correction d'atténuation et une approximation par rapport au spectre de fréquence calculé à l'étape d'analyse de fréquence de sorte qu'il existe une diminution de la contribution d'atténuation, qui se produit en raison de la profondeur de réception et de la fréquence des ondes sonores à ultrasons étant propagées, et l'extraction de données de caractéristiques du spécimen par une unité (40) d'extraction de données de caractéristiques ; et

une étape de détermination de caractérisation tissulaire incluant la détermination d'une caractérisation tissulaire d'une zone prédéterminée du spécimen en se référant à des données de caractéristiques des spectres de fréquence, chacune étant extraite sur la base des ondes sonores à ultrasons réfléchies de l'un parmi une pluralité de spécimens connus, et en se référant aux données de caractéristiques extraites à l'étape d'extraction de données de caractéristiques par une unité (43) de détermination de caractérisation tissulaire,

**caractérisé en ce que**

l'étape d'extraction de données de caractéristiques comprend soit

(i) une étape d'approximation incluant l'exécution de l'approximation par rapport au spectre de fréquence calculé par l'étape d'analyse de fréquence et l'extraction de données de caractéristiques de précorrection en tant que données de caractéristiques avant d'exécuter la correction d'atténuation, et une étape de correction d'atténuation incluant l'exécution de la correction d'atténuation par rapport aux données de caractéristiques de précorrection extraites par l'étape d'approximation, et l'extraction de données de caractéristiques du spectre de fréquence ; soit

(ii) une étape de correction d'atténuation incluant l'exécution de la correction d'atténuation par rapport au spectre de fréquence, et une étape d'approximation incluant l'exécution de l'approximation par rapport au spectre de fréquence corrigé par l'étape de correction d'atténuation et l'extraction de données de caractéristiques du spectre de fréquence.

# FIG.1

# FIG.2

# FIG.3

# FIG.4

```
         ┌─────────────┐
         │   START     │
         └─────────────┘
                │
   ┌─────────────────────────────┐
   │  MAKE MEASUREMENT OF NEW    │~S1
   │         SPECIMEN            │
   └─────────────────────────────┘
                │
   ┌─────────────────────────────┐
   │  CLASSIFY RECEIVED SIGNALS  │
   │  INTO ECHO SIGNALS FOR B-MODE│
   │  IMAGES AND ECHO SIGNALS FOR │~S2
   │  PROCESSING, AND PERFORM    │
   │  SIGNAL AMPLIFICATION       │
   └─────────────────────────────┘
                │
   ┌─────────────────────────────┐
   │  GENERATE B-MODE IMAGE DATA │~S3
   └─────────────────────────────┘
                │
   ┌─────────────────────────────┐
   │   DISPLAY B-MODE IMAGE      │~S4
   └─────────────────────────────┘
                │
            ╱S5 ╲
      ╱ IS SETTING ╲    NO
    ╱ PERFORMED FOR AREA ╲─────────────────┐
      ╲ OF CONCERN? ╱                       │
            ╲   ╱                      ╱ S7 ╲
            │YES                    ╱  END   ╲  NO
   ┌─────────────────────────────┐ ╲ OPERATIONS? ╱───┐
   │   FREQUENCY ANALYSIS        │~S6  ╲    ╱          │
   └─────────────────────────────┘     │YES           │
                │                        │             │
   ┌─────────────────────────────┐      │             │
   │  EXTRACT PRE-CORRECTION     │~S8    │             │
   │      FEATURE DATA           │       │             │
   └─────────────────────────────┘       │             │
                │                         │             │
   ┌─────────────────────────────┐       │             │
   │  PERFORM ATTENUATION        │       │             │
   │  CORRECTION OF PRE-         │~S9     │             │
   │  CORRECTION FEATURE DATA    │        │             │
   └─────────────────────────────┘        │             │
                │                          │             │
   ┌─────────────────────────────┐         │             │
   │  TISSUE CHARACTERIZATION    │~S10      │             │
   │      DETERMINATION          │          │             │
   └─────────────────────────────┘          │             │
                │                            │             │
   ┌─────────────────────────────┐           │             │
   │  GENERATE DETERMINATION-    │~S11        │             │
   │  RESULT-DISPLAY IMAGE DATA  │            │             │
   └─────────────────────────────┘            │             │
                │                              │             │
   ┌─────────────────────────────┐             │             │
   │  DISPLAY DETERMINATION RESULT│~S12         │             │
   │      DISPLAY IMAGE          │              │             │
   └─────────────────────────────┘              │             │
                │──────────────────────────────┘             │
         ┌─────────────┐                                     │
         │    END      │                                     │
         └─────────────┘                                     │
```

# FIG.5

# FIG.6

```
        ( FREQUENCY ANALYSIS )
                  │
                  ▼
         ┌─────────────────┐
         │     L=L₀        │─── S21
         └─────────────────┘
                  │
                  ▼
         ┌─────────────────┐
         │     Z=Z₀        │─── S22
         └─────────────────┘
                  │
                  ▼
         ┌─────────────────────┐
         │ OBTAIN FFT DATA GROUP│─── S23
         └─────────────────────┘
                  │
                  ▼
      ┌────────────────────────────┐
      │ IMPLEMENT WINDOW FUNCTION  │─── S24
      └────────────────────────────┘
                  │
                  ▼
              ╱╲  S25
          IS FFT          NO
      DATA GROUP ───────────────┐  S26
        NORMAL?                 ▼
            │YES        ┌──────────────────┐
            │           │ INSERT ZERO DATA │
            │           │  EQUIVALENT TO   │
            │           │     DEFICIT      │
            │           └──────────────────┘
            │◄───────────────────┘
            ▼
      ┌──────────────┐
      │  PERFORM FFT │─── S27
      └──────────────┘
            │
            ▼
      ┌──────────────┐
      │    Z=Z+D     │─── S28
      └──────────────┘
            │
            ▼
          ╱╲  S29
        Z>Zmax?  ─── NO ──┐
           │YES           │
           ▼              │
      ┌──────────┐        │
      │  L=L+1   │─── S30 │
      └──────────┘        │
           │              │
           ▼              │
   NO    ╱╲  S31          │
  ┌──── L>Lmax?           │
  │      │YES             │
  │      ▼                │
  │  ( RETURN )           │
  └──────────────────────┘
```

Step S21: $L=L_0$

Step S22: $Z=Z_0$

Step S23: OBTAIN FFT DATA GROUP

Step S24: IMPLEMENT WINDOW FUNCTION

Step S25: IS FFT DATA GROUP NORMAL?

Step S26: INSERT ZERO DATA EQUIVALENT TO DEFICIT

Step S27: PERFORM FFT

Step S28: $Z=Z+D$

Step S29: $Z>Z_{max}$?

Step S30: $L=L+1$

Step S31: $L>L_{max}$?

# FIG.7

# FIG.8

# FIG.9

# FIG.10

# FIG.11

TISSUE CHARACTERIZATION
DETERMINATION

SET FEATURE DATA SPACE — S41

CALCULATE DISTANCE IN FEATURE DATA
SPACE FROM SPECIMEN POINT TO KNOWN — S42
SPECIMEN AVERAGE POINT OF EACH GROUP

DETERMINE TISSUE CHARACTERIZATION — S43

OUTPUT DETERMINATION RESULT — S44

RETURN

# FIG.12

# FIG.13

# FIG.14

FIG.15

FIG.16

FIG.17

FIG.18

FIG.19

701 700

FIG.20

701 700

# FIG.21

```
                    ┌─────────────┐
                    │    START    │
                    └──────┬──────┘
                           ▼
        ┌──────────────────────────────┐
        │ MAKE MEASUREMENT OF NEW       │──S51
        │ SPECIMEN                      │
        └──────────────┬───────────────┘
                       ▼
        ┌──────────────────────────────┐
        │ CLASSIFY RECEIVED SIGNALS     │
        │ INTO ECHO SIGNALS FOR B-MODE  │
        │ IMAGES AND ECHO SIGNALS FOR   │──S52
        │ PROCESSING, AND PERFORM       │
        │ SIGNAL AMPLIFICATION          │
        └──────────────┬───────────────┘
                       ▼
        ┌──────────────────────────────┐
        │ GENERATE B-MODE IMAGE DATA    │──S53
        └──────────────┬───────────────┘
                       ▼
        ┌──────────────────────────────┐
        │ DISPLAY B-MODE IMAGE          │──S54
        └──────────────┬───────────────┘
                       ▼
```

S55
IS SETTING PERFORMED FOR AREA OF CONCERN?   NO

S57
END OPERATIONS?   NO

YES

FREQUENCY ANALYSIS   S56

YES

PERFORM ATTENUATION CORRECTION OF FREQUENCY SPECTRUM   S58

EXTRACT FEATURE DATA   S59

TISSUE CHARACTERIZATION DETERMINATION   S60

GENERATE DETERMINATION-RESULT-DISPLAY IMAGE DATA   S61

DISPLAY DETERMINATION RESULT DISPLAY IMAGE   S62

END

# FIG.22

# FIG.23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005122906 A
- US 2006079780 A1